# EUROPEAN PATENT APPLICATION

(11) **EP 2 813 959 A2**
(43) Date of publication of application: **17.12.2014**
(21) Application number: 14172191.0
(22) Date of filing: 12.06.2014
(51) Int. Cl.: G06F 19/00, A61B 6/00

(54) **Apparatus and method for providing medical information**

(30) Priority: 12.06.2013 KR 20130067304; 11.06.2014 KR 20140071055
(71) Applicant: Samsung Electronics Co., Ltd, Gyeonggi-do 443-742 (KR)
(72) Inventor: Ban, Dae-hyun, Seoul (KR); Lee, Woong, Gyeonggi-do (KR); Han, Jeong-ho, Seoul (KR); Oh, Keum-yong, Gyeonggi-do (KR); Lee, Chang-lae, Gyeongsangnam-do (KR)
(74) Representative: Grootscholten, Johannes A.M.

(57) **Abstract**

Provided are a medical information providing apparatus and method. The medical information providing apparatus comprises:
an image obtaining unit for capturing a shield room inside image of the shield room in which the target object is positioned;
a diagnosis information obtaining unit for obtaining diagnosis information related to a target object positioned in a shield room having a console room window for observation of the shield room;
at least one display unit for displaying the diagnostic information which is overlaid on the room image according to a first mode.

## Description

### BACKGROUND

### 1. Field

One or more embodiments of the present disclosure relate to a method and apparatus for providing medical information, which is generated by diagnosing a target object, from a medical information providing apparatus to an operator.

### 2. Description of the Related Art

A computer tomography (CT) system captures a plurality of X-ray images while the CT system rotates around one or more axes with respect to a target object, and then synthesizes the plurality of X-ray images. Since the CT system is capable of providing a cross-sectional image of the target object, the CT system may express an inner structure of the target object without an overlap therebetween, compared to a general X-ray capturing apparatus, so that the CT system is widely used.

A magnetic resonance imaging (MRI) system involves imaging information that is obtained by exposing nuclei to a magnetic field and then resonating the nuclei. The MRI apparatus is advantageous in that it is noninvasive, exhibits an excellent tissue contrast, compared to a CT apparatus, and does not generate artifacts due to bone tissue. Also, since the MRI apparatus can capture various cross-sectional images in predetermined directions without moving a target object, the MRI apparatus may be widely used with other image diagnosis apparatuses.

In regard to a general CT system or MRI system, a console room in which an operator is positioned is shielded from a shield room in which a target object is positioned. Also, a plurality of display devices and user interface devices are positioned in front of or near to the operator in the console room so as to diagnose the target object.

Thus, the operator may not efficiently use a space in the console room. Also, if an operator's view of the target object through the window is obscured (for example by the presence of a monitor, other display device or paperwork on the operator's desk) or if the operator's attention is drawn to diagnosis information shown on one or more than one of a number of display devices in front of or near to the operator, there is a risk that the operator's observation of the target object in the shield room through the window of the console room may be or become careless.

### SUMMARY

Aspects of the present disclosure include a medical information providing apparatus and a method that efficiently provide medical information by effectively disposing a plurality of display devices, as well as a non-transitory computer-readable recording medium having recorded thereon a program which, when executed by a computer, performs the medical information providing method.

Additional aspects, embodiments and/or features will be set forth in part in the description which follows and, in part, will be apparent from the description, or may be learned by practice of the presented embodiments.

According to one or more embodiments of the present specification, a medical information providing apparatus includes all features of the appended single independent apparatus claim. The display unit is preferably disposed adjacent or next to, or in or on said console room window for enlarging an observation area inside the shield room, when the shield room inside image is provided to the display unit in at least the first mode, relative to an observation area that the console room window provides when the shield room inside view is not provided to the display unit in the second mode.

According to one or more embodiments of the present disclosure, a medical information providing method is provided to include all steps according to the single appended independent method claim. The display unit is preferably disposed adjacent or next to, or in or on said console room window for enlarging an observation area inside the shield room when the shield room inside image is provided to the display unit, wherein the enlarged observation area is larger than an observation area that the console room window provides.

The medical information providing method may further include an operation of, or the apparatus may be arranged for, selecting a partial region of the shield room inside image, and an image of the partial region may be provided to the display unit.

The operation of selecting may include an operation of selecting the partial region based on at least one of a position of the display unit, a position of an operator, and a distance between the display unit and the operator.

The operation of selecting may include an operation of selecting the partial region based on at least one identification (ID) device for identifying the operator.

The operation of selecting may include an operation of selecting the partial region based on the at least one ID device of the operator that logs into a medical diagnosis system for diagnosing the target object.

The operation of determining may include an operation of determining the medical information providing mode while a diagnosis process with respect to the target object is being performed.

The operation of determining may include an operation of changing the first mode to the second mode or the third mode, when the diagnosis process with respect to the target object starts.

When a plurality of the display units exist, the operation of determining may include an operation of determining the medical information providing mode of each of the plurality of the display units.

When a number of pieces of obtained diagnosis information are increased, the operation of determining may include an operation of determining the medical information providing mode of each of the plurality of the display units as the second mode or the third mode.

The operation of determining may include an operation of changing the medical information providing mode of each of the plurality of the display units, according to priority orders that are pre-set in the plurality of the display units.

The priority orders may be determined based on at least one of a position, a size, and a resolution of each of the plurality of the display units.

When a number of pieces of obtained diagnosis information is increased, the providing may include an operation of providing medical information, which has been provided to a first display unit, to a second display unit that is lower, in terms of the priority orders, than the first display unit.

The plurality of the display units may be disposed adjacent to each other along a side edge of the console room window.

The medical information providing method may further include operations of, or the apparatus may be arranged for, selecting a plurality of partial regions from the shield room inside image according to an arrangement of the plurality of the display units; and matching the plurality of partial regions with the plurality of the display units, respectively, and images of the plurality of partial regions may be provided to the plurality of the matched display units.

The plurality of partial regions may be spatially connected to each other.

The medical information providing method may further include operations of, or the apparatus may be arranged for, receiving an external input signal via a user input unit that commonly corresponds to the plurality of the display units; and controlling the plurality of the display units based on the external input signal.

In the third mode, the diagnosis information may be overlaid on the shield room inside image.

The diagnosis information may include at least one of ID information of the target object, information about a diagnosis process with respect to the target object, and diagnosis history information of the target object.

A medical diagnosis system for diagnosing the target object may include a magnetic resonance imaging (MRI) system or a computer tomography (CT) system.

According to the above referred aspect embodiments of the present disclosure, there is provided a non-transitory computer-readable recording medium having recorded thereon a program which, when executed by a computer, performs the method.

One or more exemplary embodiments provide a medical information providing method and apparatus therefor that efficiently provide medical information by efficiently disposing a plurality of display devices.

One or more embodiments also provide a non-transitory computer-readable recording medium having recorded thereon a program which, when executed by a computer, performs the medical information providing method.

According to an aspect of an exemplary embodiment, a medical information providing method includes operations of capturing a shield room inside image of a shield room in which an object is positioned; obtaining diagnostic information related to the object; determining a medical information providing mode as one of a first mode for providing the shield room inside image, a second mode for providing the diagnostic information, and a third mode for providing the shield room inside image and the diagnostic information; and providing at least one of the shield room inside image and the diagnostic information to a display, according to the medical information providing mode, wherein the display is disposed adjacent to a console room window for observation of the shield room.

The medical information providing method may further include an operation of selecting a partial region of the shield room inside image, and an image of the partial region may be provided to the display.

The operation of selecting may include an operation of selecting the partial region based on at least one of a position of the display, a position of an operator, and a distance between the display and the operator.

The operation of selecting may include an operation of selecting the partial region based on at least one identification (ID) device for identifying the operator.

The operation of selecting may include an operation of selecting the partial region based on the at least one ID device of the operator that logs into a medical diagnostic system for diagnosing the object.

The operation of determining may include an operation of determining the medical information providing mode while a diagnostic process with respect to the object is being performed.

The operation of displaying may include operations of displaying the room image according to the first mode on the display; changing the first mode to the second mode or the third mode, when the diagnostic process with respect to the object starts; and displaying the diagnostic information in the second mode or the third mode based on a mode change, on a same displays.

The operation of determining may include an operation of changing the first mode to the second mode or the third mode, when the diagnostic process with respect to the object starts.

The operation of displaying may include changing the medical information providing mode of the plurality of displays, based on priority orders pre-set in respective displays of the plurality of displays.

The priority orders may be determined based on at least one of a position, a size, and a resolution of the respective displays.

The respective displays may include a first display and a second display. And, the operation of displaying may include displaying a first piece of the diagnostic information on the first display, when a diagnostic process of the object begins; consequently, displaying the first piece of the diagnostic information on the second display that has a lower priority than that of the first display, when the diagnostic process of the object progresses and a second piece of the diagnostic information is obtained; and displaying the second piece of the diagnostic information on the first display.

The display panel may be embodied in a console room window, and the plurality of displays is disposed adjacent each other one of along a side edge of the console room window and on an entirety of the console room window.

The medical information providing method may further include selecting a plurality of partial regions from the room image according to an arrangement of the plurality of displays; matching the plurality of partial regions with the plurality of displays, respectively; and displaying images of the plurality of partial regions on the plurality of the matched displays.

The plurality of partial regions may be spatially connected to each other.

The medical information providing method may further include receiving an external input signal via a user input unit that commonly corresponds to the plurality of displays; and controlling the plurality of displays based on the external input signal.

In the third mode, the diagnostic information may be overlaid on the room image.

The diagnostic information may include at least one of: an identification (ID) information of the object, information about a diagnostic process with respect to the object, and medical history information of the object.

A magnetic resonance imaging (MRI) system or a computed tomography (CT) system which performs the medical information providing methods.

According to an aspect of an exemplary embodiment, a medical information providing apparatus includes an image obtainer configured to capture a room image of an inside of an examination room in which an object is positioned for medical examination; a diagnostic information obtainer configured to obtain diagnostic information of the object; a mode determiner configured to define a medical information providing mode to include: a first mode configured to provide the room image, a second mode configured to provide the diagnostic information, and a third mode configured to provide the diagnostic information on the room image; and a controller configured to provide at least one of the room image and the diagnostic information, according to the medical information providing mode, to a display disposed at a display panel positioned in a console room which is separated from the examination room.

The medical information providing apparatus further includes an image processor configured to select a partial region of the room image, wherein an image of the partial region is displayed on the display.

The image obtainer may select the partial region based on at least one of: a position of the display, a position of an operator, and a distance between the display and the operator.

The image obtainer may select the partial region based on an identification (ID) device configured to identify the operator.

The image obtainer may select the partial region based on the ID device of the operator that logs into a medical diagnostic system to perform the medical examination of the object.

The mode determiner may be configured to determine the medical information providing mode for the display while a diagnostic process with respect to the object is being performed.

The room image according to the first mode may be displayed on the display, prior to beginning of the diagnostic process, the mode determiner may change the first mode to the second mode or the third mode, when the diagnostic process with respect to the object starts, and the display may display the diagnostic information in the second mode or the third mode based on a mode change.

The medical information providing apparatus may further include a plurality of displays, wherein the mode determiner determines the medical information providing mode of the plurality of displays.

The mode determiner may determine the medical information providing mode of the plurality of displays as the second mode or the third mode, when a diagnostic process of the object begins.

The mode determiner may change the medical information providing mode of the plurality of displays, based on priority orders pre-set in respective displays of the plurality of displays.

The priority orders may be determined based on at least one of a position, a size, and a resolution of the respective displays.

The respective displays may include a first display and a second display, and the controller may provide a first piece of the diagnostic information to the first display, when a diagnostic process of the object begins, consequently provides the first piece of the diagnostic information to the second display that has a lower priority than that of the first display, when the diagnostic process of the object progresses and a second piece of the diagnostic information is obtained, and provide the second piece of the diagnostic information to the first display.

The display panel may be embodied in a console room window, and the plurality of displays may be disposed adjacent each other one of along a side edge of the console room window and an entirety of the console room window.

The image processor selects a plurality of partial regions from the room image according to an arrangement of the plurality of displays, matches the plurality of partial regions with the plurality of displays, respectively, and provides images of the plurality of partial regions to the plurality of matched displays.

The plurality of partial regions may be spatially connected to each other.

The medical information providing apparatus may further include user input unit that commonly corresponds to the plurality of displays, wherein the controller may control the plurality of displays based on an external input signal that is received via the user input unit.

In the third mode, the diagnostic information may be overlaid on the room image.

The diagnostic information may include at least one of: an identification (ID) information of the object, information about a diagnostic process with respect to the object, and medical history information of the object.

The medical information providing apparatus may further include a magnetic resonance imaging (MRI) system or a computed tomography (CT) system.

According to an aspect of an exemplary embodiment, an apparatus includes image obtainers configured to capture a room image of an inside of a first room, in which an object is positioned for medical imaging; a room image processor configured to obtain the room image from the image obtainers and form a visual representation of the room image; a medical processor configured to process medical imaging information of the object while the medical imaging of the object proceeds and form a medical image of a region of interest (ROI) of the object; and displays which are disposed in an array on a display panel in a second room separated from the first room and configured to display at least one of the visual representation of the room image and the medical image of the ROI of the object.

The apparatus may further include a user workstation which is disposed in the second room and comprises a user interface (UI) configured to interface with the displays. Wherein a user can manipulate one of graphical objects displayed on the UI and physical keys provided with the UI to control the displays.

The room image processor and the medical processor may embody in the workstation.

The apparatus may further include a mode determiner configured to determine a medical information providing mode in the displays to include at least one of: a first mode configured to provide the room image, a second mode configured to provide the medical image, and a third mode configured to provide the medical image overlaid on the room image.

The image obtainers can be attached to rear surfaces of corresponding displays.

The image obtainers can be configured to obtain portions of the room image corresponding to a field of view (FOV) of respective image obtainers, the room image processor may be configured to provide the portions of the room image to the displays which have been configured to provide the room image, and the medical processor can be configured to provide the medical image of the object on at least one of the room image and the displays configured to provide the medical image.

The displays may be attached to the display panel side by side to form a seamless display comprising display tiles arranged in addressable array.

According to an aspect of an exemplary embodiment, an apparatus includes an image obtainer configured to obtain a room image of an inside of a first room, in which an object is positioned for medical imaging; a medical processor configured to obtain pieces of medical information of the object; displays which are disposed in an array on a display panel in a second room; and a user workstation which is disposed in the second room and configured to control the medical imaging of the object and display at least one of the room image and the pieces of medical information on the displays.

The medical processor may include an imaging processor configured to obtain a medical image of the object and provide the medical image to the displays; a pre-processor configured to obtain and provide an imaging schedule of the object to the displays; and a contrast monitoring processor configured to control an injection of contrast in the object and provide a contrast monitoring image to the displays.

The workstation may be configured to define an image providing mode and set an image providing order in the displays, and to control a display of the room image, the medical image, the imaging schedule, and the contrast monitoring image based on the defined image providing mode and the set image providing order, on respective displays.

The apparatus may further include at least one of a computed tomography (CT) imaging system and a magnetic resonance (MR) imaging system, wherein the workstation is configured to provide at least one of the CT image and the MR image to the displays.

The medical processor may include a registration processor configured to superimpose a CT image of a region of interest (ROI) of the object and an MR image of the ROI of the object, and the workstation is configured to provide a superimposed image to the displays.

### BRIEF DESCRIPTION OF THE DRAWINGS

These and/or other aspects will become apparent and more readily appreciated from the following description of the embodiments, taken in conjunction with the accompanying drawings of which:
FIG. 1 illustrates general technologies related to embodiments of the present disclosure;
FIG. 2 illustrates a relation between a medical information providing apparatus, a console room window, and a medical diagnosis system, according to an embodiment of the present disclosure;
FIG. 3 illustrates a medical information providing mode of a display unit, according to an embodiment of the present disclosure;
FIG. 4 is a block diagram of the medical information providing apparatus, according to an embodiment of the present disclosure;
FIG. 5 is a flowchart of a medical information providing method, according to an embodiment of the present disclosure;
FIG. 6 is a flowchart of a medical information providing method, according to another embodiment of the present disclosure;
FIG. 7 illustrates operations between the medical information providing apparatus and the medical diagnosis system, according to an embodiment of the present disclosure;
FIG. 8 illustrates operations between the medical information providing apparatus and the medical diagnosis system, according to another embodiment of the present disclosure;
FIG. 9 illustrates an example in which the medical information providing apparatus senses an operator, according to an embodiment of the present disclosure;
FIG. 10 is a flowchart of a method of selecting a partial region of an image of an inside of a shield room (hereinafter referred to as a "a shield room inside image"), according to an embodiment of the present disclosure;
FIG. 11 illustrates a method of selecting, by the medical information providing apparatus, a partial region of a shield room inside image according to an operator's view, according to an embodiment of the present disclosure;
FIG. 12 is a flowchart of a method, performed by the medical information providing apparatus, of capturing shield room inside images by using a plurality of image obtaining units, and outputting the shield room inside images to a plurality of display units, according to an embodiment of the present disclosure;
FIGS. 13A and 13B illustrate image obtaining units capturing shield room inside images, according to embodiments of the present disclosure;
FIG. 14 illustrates an example in which the medical information providing apparatus outputs a shield room inside image to a plurality of display units, according to an embodiment of the present disclosure;
FIG. 15 illustrates an example in which the medical information providing apparatus provides medical information during a second mode or a third mode, according to an embodiment of the present disclosure;
FIG. 16 illustrates a method of changing a medical information providing mode, by the medical information providing apparatus, when a diagnosis process with respect to a target object is performed, according to an embodiment of the present disclosure;
FIG. 17 illustrates priority orders that are set in the display units included in the medical information providing apparatus, according to an embodiment of the present disclosure;
FIG. 18 illustrates an example in which the medical information providing apparatus outputs diagnosis information according to priority orders of display units, according to an embodiment of the present disclosure;
FIG. 19 illustrates an example in which the medical information providing apparatus outputs diagnosis information to display units according to importance of the diagnosis information, according to an embodiment of the present disclosure; and
FIG. 20 illustrates a user input unit that commonly corresponds to a plurality of display units, according to an embodiment of the present disclosure;
FIGS. 21A, 21B, 21C, and 21D illustrate exemplary display arrangements;
FIG. 22 illustrates a medical information providing system, according to an exemplary embodiment;
FIG. 23 illustrates a detail of a medical information providing system, according to an exemplary embodiment; and
FIG. 24 illustrates a detail of a medical information providing system, according to an exemplary embodiment.

### DETAILED DESCRIPTION

All terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

Also, when a part "includes" or "comprises" an element, unless there is a particular description contrary thereto, the part may further include other elements, not excluding the other elements. In the following description, terms such as "unit" may be embodied as, but not limited to, software or a hardware component, such as a field programmable gate array (FPGA) or application specific integrated circuit (ASIC). However, a unit may advantageously be configured to reside on an addressable storage medium and configured to execute on one or more processors. Thus, a unit may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. The functionality provided for in the components and units may be combined into fewer components and units or further separated into additional components and units.

Throughout the specification, an "image" may mean multidimensional data formed of discrete image elements (e.g., pixels of a two-dimensional (2D) image and voxels of a three-dimensional (3D) image). For example, the image may include a medical image of a target object which is obtained by using an X-ray, computer tomography (CT), magnetic resonance imaging (MRI), an ultrasonic wave, or other medical diagnosis systems.

Also, throughout the specification, a "target object" may include a human, an animal, or a part of a human or animal. For example, the target object may include organs such as liver, heart, womb, brain, breast, abdomen, or the like, or a blood vessel, or a fetus or nuchal translucency (NT). Also, the target object may include a phantom. The phantom means a material having a volume that is very close to a density and effective atomic number of an organism, and may include a sphere phantom having a characteristic similar to a physical body.

Throughout the specification, a "user" may be, but is not limited to, a medical expert including a doctor, a nurse, a medical laboratory technologist, a medial image expert, a radiologist, and a technician who repairs a medical apparatus.

Examples of specific, non-limiting embodiments will be described below more fully, with reference to the accompanying drawings. The embodiments may, however, be embodied in many different forms and should not be construed as being limited to the embodiments set forth herein; rather, these embodiments are provided so that this disclosure will be thorough and complete, and will fully convey the concept of these embodiments to those of ordinary skill in the art. In the following description, well-known functions or constructions are not described in detail since they would obscure the specification with unnecessary detail. Throughout the following description, like reference numerals in the drawings may denote the same, alike or just similar elements.

Expressions such as "at least one of," when preceding a list of elements, modify the entire list of elements and do not modify the individual elements of the list.

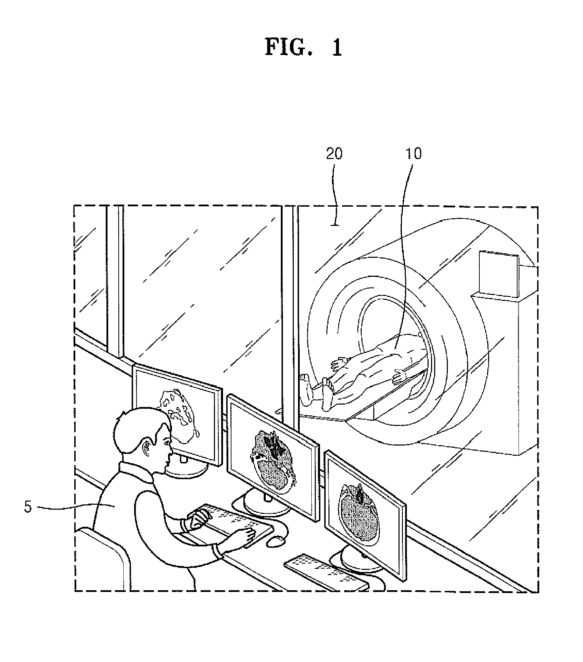

FIG. 1 illustrates general technologies related to embodiments of the present invention.

In a medical diagnosis system such as an MRI system, a CT system, an X-ray system, or the like, an operator 5 in a console room diagnoses a target object 10 in a shield room. The console room and the shield room are separated from each other by a shield wall so as to protect the operator 5 and/or the operator's equipment from a magnetic field, radiation, a radio frequency (RF) signal, or the like.

The operator 5 of the medical diagnosis system has to directly observe the inside of the shield room and the target object 10 while diagnosing the target object 10. That is, the operator 5 has to directly check various factors such as a position of the target object 10 on a diagnosis table, movement of the target object 10 during diagnosis, or the like that are related to the target object 10 in the shield room. For this reason, the shield wall that separates the console room and the shield room may include a console room window 20.

The console room window 20 is a unit that is used by the operator 5 in the console room so as to observe the target object 10 in shield room. The operator 5 may diagnose the target object 10 using presented information that is displayed on monitors, user interfaces or other display devices in front of or near to the operator 5, while the operator 5 also needs to directly observe the target object 10 and the inside the console room via the console room window 20 that is transparent or translucent.

As illustrated in FIG. 1, the operator 5 may use a plurality of user interfaces so as to diagnose the target object 10. The operator 5 may diagnose the target object 10 by controlling a diagnosis process and obtaining a medical image via a plurality of input units and output units that are related to the medical diagnosis system.

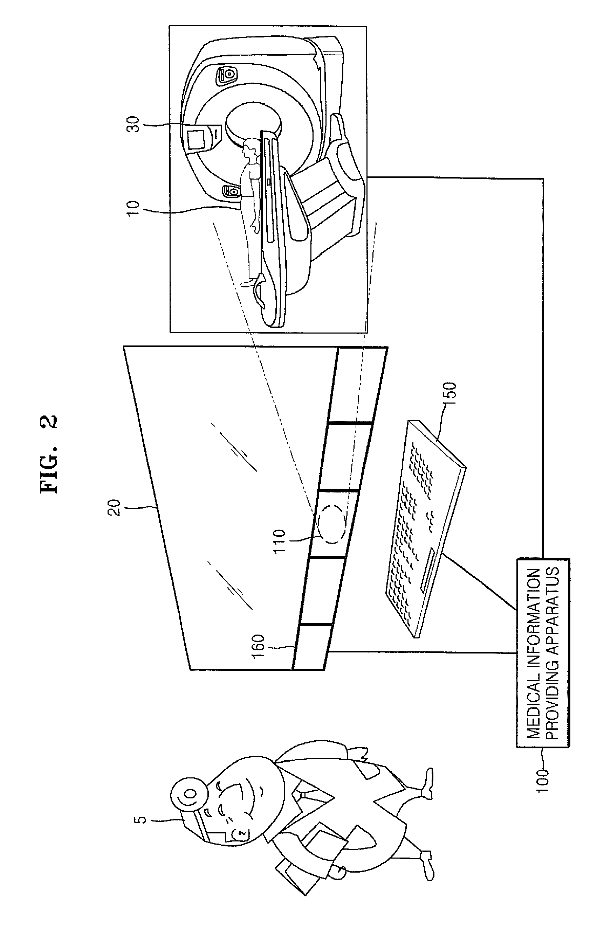

FIG. 2 illustrates a relation between a medical information providing apparatus 100, the console room window 20, and a medical diagnosis system 30, according to an embodiment of the present invention.

As described above with reference to FIG. 1, the operator 5 of the medical information providing apparatus 100 in the console room may observe the target object 10 in the shield room and may also observe operation of the medical diagnosis system 30, both through the transparent console room window 20.

The medical information providing apparatus 100 shown in FIG. 2 may be connected to the medical diagnosis system 30, so that the medical information providing apparatus 100 may receive diagnosis information and may display the diagnosis information via a display unit 160. In the present embodiment, the medical information providing apparatus 100 may include an image obtaining unit 110 for capturing an image of the inside of a shield room (hereinafter referred to as a "a shield room inside image"), and may display the shield room inside image via the display unit 160. Additionally or alternatively, at least one image obtaining unit 110, such as a camera, may be disposed within the shield room and separate from the display units 160. Thus, a view of the inside of the shield room offered to the operator 5 is enlarged relative to a size of the window 20 defined by a boundary of the window, when the display unit 160 are arranged next to or outside said physical boundary of the window 20 and operate in the first mode to show the shield room inside image, or in the third mode with a transparent second mode with shield room inside image of the first mode in the background on the display unit. However, such a camera inside the shield room may need to be protected against influence from a magnetic field, radiation, a radio frequency (RF) signal, or the like.

That is, the medical information providing apparatus 100 may provide, to the operator 5, at least one of the shield room inside image and the diagnosis information via the display unit 160. An embodiment in which the medical information providing apparatus 100 provides information of a target object or medical information, i.e. the at least one of the shield room inside image and the diagnosis information, to the operator 5 will be described at a later time.

The "diagnosis information" may indicate information to be used in diagnosing the target object 10, and may include various types of information that are related to the target object 10 and that are obtained via the medical diagnosis system 30 or from elsewhere, such as an administrative server. For example, the diagnosis information may include pre-stored information that is related to the target object 10 and includes identification (ID) information such as a patient's hospital ID to identify the target object 10, patient information such as an age, a name, a gender, or the like of the target object 10, information about a medical image capturing history of the target object 10, a patient list indicating a medical image capturing schedule, or the like.

In addition, the diagnosis information may also include information about a movement path of a diagnosis table on which the target object 10 is positioned, a medical image that is a result of diagnosing the target object 10, an image of a path along which a contrast medium that is injected into the target object 10 spreads, and information about a progress of a protocol that diagnoses the target object 10. However, the diagnosis information is not limited to the aforementioned information but may include all types of information related to diagnosis of the target object 10 and/or to the target object itself (age, gender, etc).

The operator 5 of the medical information providing apparatus 100 may observe the inside of the shield room via the console room window 20 and may simultaneously receive the medical information from the medical information providing apparatus 100. That is, due to the display unit 160 that is disposed around the console room window 20, the operator 5 may observe and may simultaneously diagnose the target object 10 without disruption of his/her view.

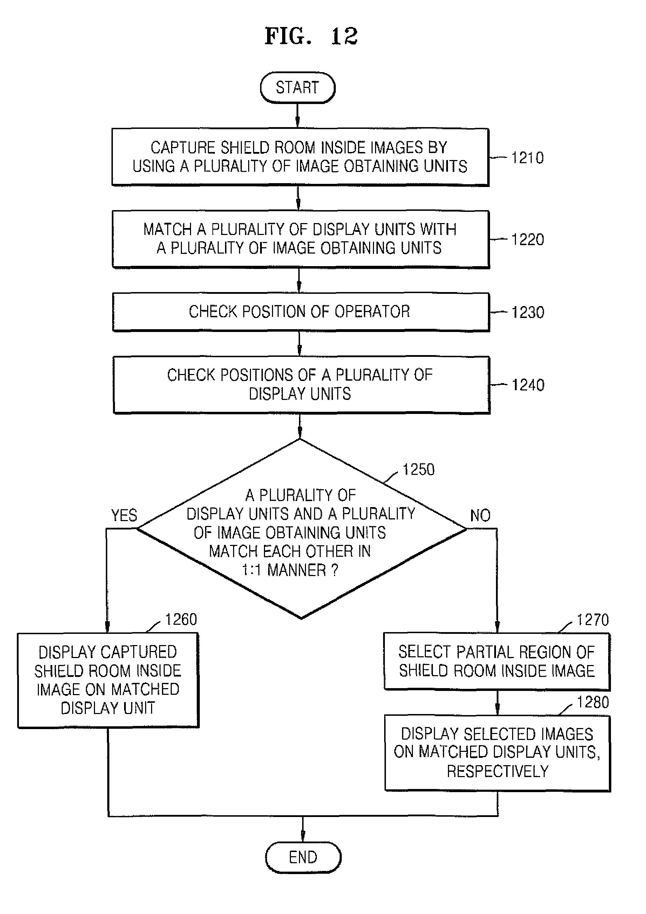

A number of the image obtaining unit 110 and the display unit 160, and their positions on the console room window 20 shown in FIG. 2 are not limited to those presented in figure 2. A detailed embodiment about the positions of the image obtaining unit 110 and the display unit 160 will be described later with reference to FIGS. 12 through 21.

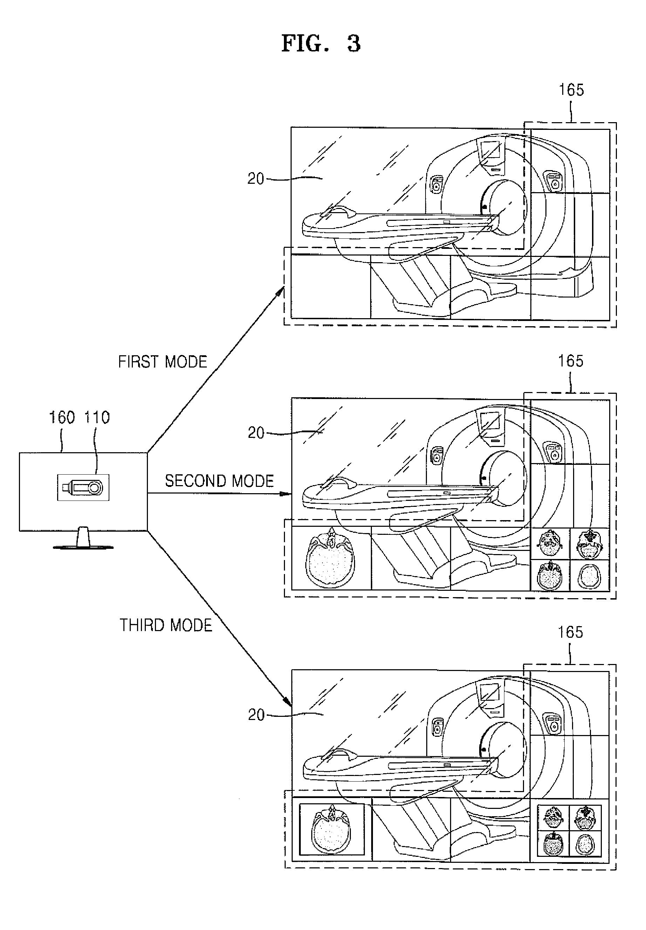

FIG. 3 illustrates a medical information providing mode of the display unit 160, according to an embodiment of the present invention.

The medical information providing apparatus 100 may determine a medical information providing mode of the display unit 160 so as to provide medical information. Here, the "medical information providing mode" means a type of medical information that is output by the medical information providing apparatus 100 via the display unit 160. The medical information providing apparatus 100 may output the medical information including at least one of a shield room inside image and the diagnosis information, and the medical information providing mode may vary according to a type of the medical information.

For example, a first mode may indicate a case in which the medical information providing apparatus 100 provides the shield room inside image to the display unit 160, a second mode may indicate a case in which the medical information providing apparatus 100 provides the diagnosis information, which is obtained from the medical diagnosis system 30, via the display unit 160, and a third mode may indicate a case in which the medical information providing apparatus 100 provides both the shield room inside image and the diagnosis information. Medical information providing mode may include at least one selected from the first mode, the second mode, and the third mode. Hereinafter, the respective first, second, and third modes of the medical information providing mode are described in detail.

First, regarding the first mode, the display unit 160 and the image obtaining unit 110 are described below. The medical information providing apparatus 100 includes a plurality of the display units 160 that are disposed adjacent to the console room window 20. The display units 160 are shown with a dotted line 165 in FIG. 3. As illustrated in FIG. 2, an arrangement and positions of the display units 160 are not limited to FIG. 3.

The medical information providing apparatus 100 may include the image obtaining unit 110 at a back side of the display unit 160. The image obtaining unit 110 may capture a shield room inside image, and the medical information providing apparatus 100 may include a plurality of the image obtaining unit 110 that correspond to the plurality of the display units 160, respectively. That is, the medical information providing apparatus 100 may include the image obtaining unit 110 at a back side of each of the display units 160.

The medical information providing apparatus 100 may include one or more display units 160.

On the other hand, in another embodiment, the medical information providing apparatus 100 may include the image obtaining units 110 that correspond to some of the display units 160. That is, in the embodiment shown in FIG. 3, the image obtaining units 110 may be arranged at back sides of only four of the display units 160 that are horizontally arranged from among six of the display units 160 marked by dotted line 165. Alternatively, the image obtaining unit 110 may be disposed at a back side of only one display unit 160 from among six of the display units 160.

In the first mode, the medical information providing apparatus 100 provides, to the display unit 160, the shield room inside image which is captured by the image obtaining unit 110. That is, the medical information providing apparatus 100 sets a virtual region inside the shield room that the operator 5 can view with the naked eye via a space in which the specific display unit 160 is disposed, and then sets a partial or entire region that corresponds to the virtual region of the shield room inside image. Afterward, the medical information providing apparatus 100 may provide a selected region to the specific display unit 160.

In the example shown at an upper side in FIG. 3, all six of the display units 160 that are marked by the dotted line 165 display the shield room inside image which is captured while these six display units 160 operate in the first mode.

The medical information providing apparatus 100 captures and provides the shield room inside image via image obtaining unit 110 associated with the display unit 160 that are disposed adjacent to the console room window 20. Accordingly, the medical information providing apparatus 100 may generate an effect by which the console room window 20 for observation inside the shield room is enlarged.

Next, the second mode is described below. In the second mode, the medical information providing apparatus 100 obtains diagnosis information and outputs the diagnosis information via the display unit 160. The medical information providing apparatus 100 may provide, to the display unit 160, various types of diagnosis information including a medical image, a spread contrast medium image, a medical image capturing schedule list, or the like that are described with reference to FIG. 2.

Referring to FIG. 1, a plurality of output devices (such as monitors, or the like) are separate from the console room window 20 in a console room according to the related art. Thus, the operator 5 has difficulty in checking an image output on the monitors while the operator 5 observes the target object 10 through the console room window 20.

On the other hand, the medical information providing apparatus 100 that operates in the second mode according to the present embodiment outputs and displays the diagnosis information via the display units 160 that are disposed adjacent to the console room window 20, so that disruption of a view of the operator 5 on the apparatus 30 and/or the target object 10 may be minimized while the target object 10 is being diagnosed.

In the example shown in the middle in FIG. 3, two of the display units 160 at lower left and lower right sides from among six of the display units 160 that are marked by the dotted line 165 may operate in the second mode. That is, the two display units 160 may obtain, as the diagnosis information, a medical image of the target object 10, and may output and display the medical image. The other four display units 160 within the dotted line may operate in the first mode and may output and display the shield room inside image.

Now, the third mode will be described below. In the third mode, the medical information providing apparatus 100 provides, to the display unit 160, all of the shield room inside image and diagnosis information that is obtained for example from the medical diagnosis system 30, a patient administration server or the like.

In the present embodiment, the medical information providing apparatus 100 may overlay the diagnosis information on the shield room inside image and may output them. In another embodiment, the medical information providing apparatus 100 may adjust transparency of the diagnosis information that is overlaid on the shield room inside image and may output them together on the display unit 160.

In the example shown at a lower side in FIG. 3, two of the display units 160 from among six of the display units 160 that are at lower left and lower right sides relative to the window operate in the third mode. That is, the two display units 160 display both the shield room inside image and the diagnosis information, and as illustrated in FIG. 3, may overlay the diagnosis information on the shield room inside image, and may display the composite image or view of the shield room inside image or view as well and the diagnosis information.

The medical information providing apparatus 100 may adjust a size of the diagnosis information. That is, in the example of the third mode shown in FIG. 3, the medical information providing apparatus 100 may adjust the size of the diagnosis information that is overlaid on the shield room inside image and may display them. This scaling of a region for display of the diagnosis information to within the circumference of the display device 160 may also be effected in the second mode. Even if the display is then set to function in the second mode (to show only the diagnosis information without transparency), a free peripheral edge surrounding diagnosis information can still be used to represent an additional portion of the shield room inside image or view. This could be referred to as the third mode, since both the shield room inside view or image and the diagnosis information are represented but without transparency of the diagnosis information. However, such a possibility could also be referred to as a second mode embodiment (because the diagnosis information is not transparent) with scaling of the diagnosis information and filling of a border edge with a portion of the shield room inside image or view. This goes to show that the skilled person should not interpret these modes described above to be strictly separate and divided, and some overlaps in modes may exist.

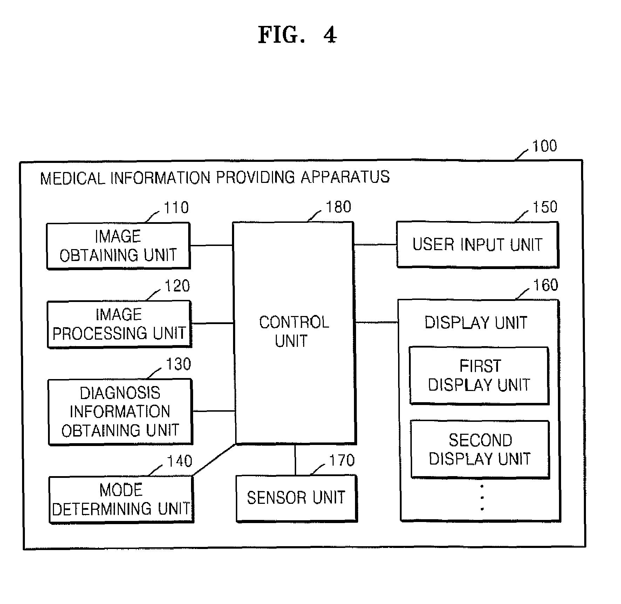

FIG. 4 is a block diagram of the medical information providing apparatus 100, according to an embodiment. The medical information providing apparatus 100 may include the image obtaining unit 110, a diagnostic information obtaining unit 130, and the display unit 160. The medical information providing apparatus 100 may further include an image processing unit 120, a mode determining unit 140, a user input unit 150, a sensor unit 170, and a control unit 180. However, not all shown elements are necessary elements. That is, the medical information providing apparatus 100 may be embodied with more or less elements than the shown elements.

Hereinafter, the aforementioned elements will be described.

The image obtaining unit 110 captures a room image of an inside of an examination room in which an object is positioned for medical examination. In detail, the image obtaining unit 110 captures a shield room inside image of the shield room in which the target object 10 is positioned. A position and region inside the shield room that the image obtaining unit 110 captures may be adjusted by the operator manipulating the user input unit 150 or by the control unit 180. For example, the image obtaining unit 110 may capture a specific position or region inside the shield room, based on a user input that is received by the user input unit 150.

The diagnostic information obtaining unit 130 obtains diagnostic information of the object.

The display unit 160 displays the diagnostic information which is overlaid on the room image according to the third mode.

In another embodiment, the medical information providing apparatus 100 may include one or more image obtaining units 110. In the present embodiment, the medical information providing apparatus 100 may include a plurality of the image obtaining units 110 that correspond to the display units 160, respectively. In another embodiment, the medical information providing apparatus 100 may be configured so that one image obtaining unit 110 corresponds to at least two display units 160. Then, the image obtaining unit 110 of or for the two display units 160 may be associated with one of the two display units 160, with which the image obtaining unit 110 is associated. Embodiments of the present disclosure encompass configurations with as many image obtaining units 110 as display units 160, or less or more. The image obtaining units 110 are each associated with one display unit in the above described embodiment, but more than one display unit 160 can be associated with a single image obtaining unit 110 and the image obtaining unit 110 can be arranged separate from the display units 160, in particular inside the shield room or behind a small window between the console room and the shield room. In particular such an embodiment with camera's separate from the display units allows the display units to be arranged on a wall next or adjacent to the physical boundaries of the window, to allow an operator to observe a larger observable area, than the area that could have been observed through the window alone.

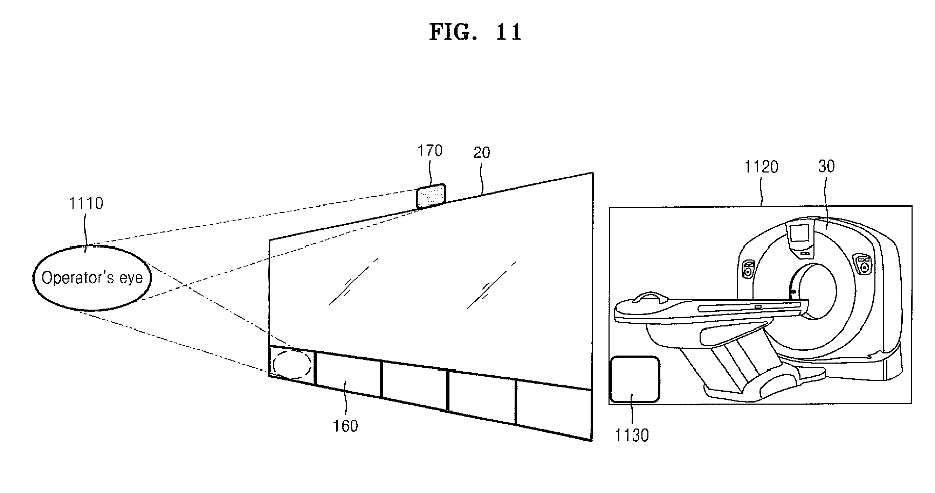

That is, the embodiments shown in FIGS. 2 and 3, the medical information providing apparatus 100 includes one image obtaining unit 110, but embodiments of the present disclosure are not necessarily limited thereto; the medical information providing apparatus 100 may include a plurality of the image obtaining units 110 as in embodiments to be described later with reference to FIGS. 11 through 13.

In the present embodiment, the image obtaining unit 110 may be disposed at a back side of the display unit 160. When the medical information providing apparatus 100 includes one image obtaining unit 110, as illustrated in FIG. 2, the image obtaining unit 110 may be attached at a center of the back side of the display unit 160 and may capture the shield room inside image through the window 20.

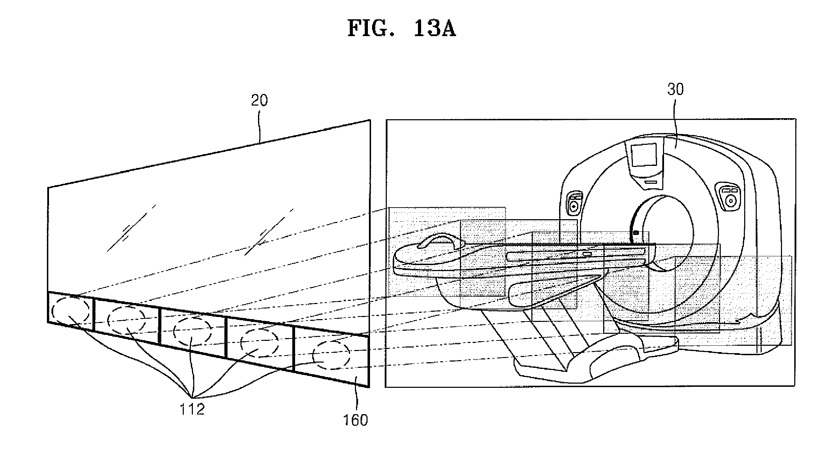

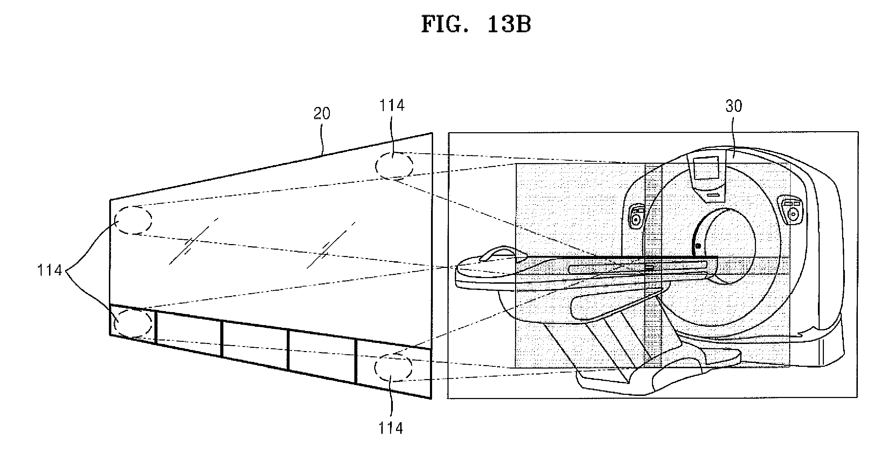

In another embodiment, when the medical information providing apparatus 100 includes a plurality of the image obtaining units 110, as illustrated in FIG. 13a, the image obtaining units 110 may be disposed at several positions at a back side of the console room window 20. For example, the image obtaining units 110 may be respectively disposed at back sides of the display units 160 that are arrayed in one direction at a lower end of the console room window 20. In another embodiment, the image obtaining unit 110 may be disposed at one or several corners of the console room window 20, see FIG 13b, even in corners where no display unit 160 is arranged, i.e. separate from the backsides of the display units 160. However, embodiments of the present disclosure are not limited to the embodiments of FIG. 13a and b; thus, the image obtaining unit 110 may be disposed in various arrangements and may capture the shield room inside image.

The image obtaining unit 110 may include various image-capturing apparatuses such as an infrared camera, a high-speed camera, a wide viewing angle camera, or the like, and may obtain a still image and a moving picture of the inside of the shield room.

The image processing unit 120 generates a shield room inside image from a resultant image obtained by the image obtaining unit 110 that captures an image inside the shield room. The image processing unit 120 may generate an image to be displayed on the display unit 160, by using the generated shield room inside image.

That is, the image processing unit 120 may select a partial or entire region of the shield room inside image. For example, when the medical information providing apparatus 100 includes a smaller number of the image obtaining units 110 than a number of the display units 160, the image processing unit 120 may select a partial region of the shield room inside image that is captured by the image obtaining units 110, wherein an image of the partial region is to be displayed on the display units 160.

In another embodiment, when the medical information providing apparatus 100 includes the image obtaining units 110 that correspond to the display units 160, respectively, the image processing unit 120 may generate the shield room inside image and may select an entire region of the shield room inside image as a region to be displayed on the display units 160. On the other hand, the image processing unit 120 may select a partial region by editing or adjusting the shield room inside image.

In the present embodiment, the image processing unit 120 may select a partial region that is of the shield room inside image and that is to be provided to the display unit 160, according to various references. For example, the image processing unit 120 may select the partial region, based on a position of the display unit 160 that is to display the shield room inside image and that is on the console room window 20. That is, in a case of the display unit 160 that is disposed at a lower right corner on the console room window 20, the image processing unit 120 may select a lower right partial region of the shield room inside image.

The image processing unit 120 may select a partial region, in consideration of an arrangement of the display units 160. That is, when the display units 160 are horizontally arrayed in one direction, the image processing unit 120 may horizontally divide the shield room inside image and then may select a plurality of partial regions. On the other hand, as illustrated in FIG. 3, when the display units 160 are arrayed in an "L"-shape, the image processing unit 120 may divide the shield room inside image into the "L"-shape and then may select a plurality of partial regions.

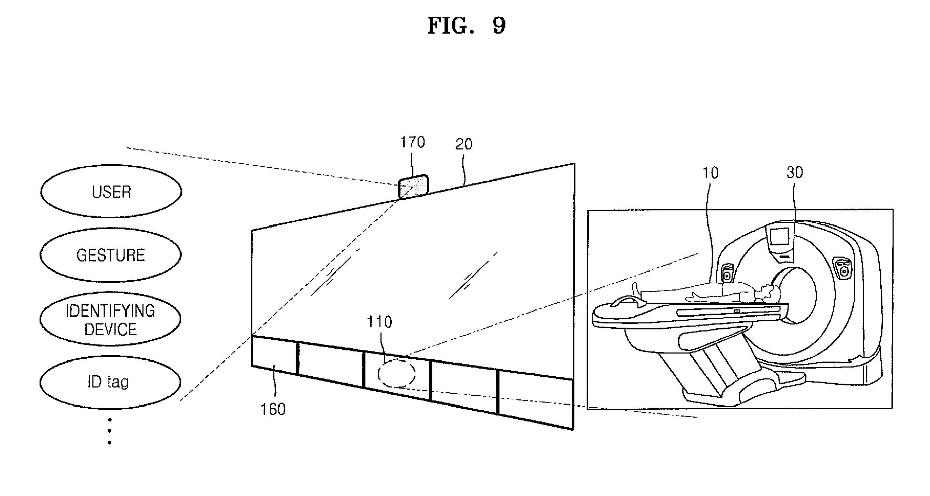

In another embodiment, the image processing unit 120 may detect a current position of the operator 5 and may select a partial region according to the position of the operator 5. That is, when the operator 5 is positioned at a right side with respect to a center of the console room window 20, the image processing unit 120 may select the partial region by referring to a direction of a view of the operator 5 with respect to the console room window 20. This will be described in detail later with reference to FIGS. 9 through 11.

However, one or more embodiments of the present disclosure are not limited to the aforementioned embodiments. That is, the image processing unit 120 may select a partial or entire region of the shield room inside image according to various references. Also, the image processing unit 120 may select a region by simultaneously referring to several references, instead of just one reference.

The diagnosis information obtaining unit 130 obtains diagnosis information related to the target object 10. As illustrated in FIG. 2, the diagnosis information may include various types of information that are used in diagnosing the target object 10.

The diagnosis information obtaining unit 130 may obtain the diagnosis information from the medical diagnosis system 30 or an external server. For example, the diagnosis information obtaining unit 130 may obtain a medical image that is a result obtained by diagnosing the target object 10, as the diagnosis information, from the medical diagnosis system 30. In another embodiment, the diagnosis information obtaining unit 130 may obtain the diagnosis information about personal information or a medical image capturing schedule of a patient from a hospital server.

The diagnosis information obtaining unit 130 may communicate with the medical diagnosis system 30 or the external server via a wireless or wired network, so that the diagnosis information obtaining unit 130 may obtain the diagnosis information. In the present embodiment, the diagnosis information obtaining unit 130 may exchange data with the hospital server or other medical apparatuses in a hospital connected via a Picture Archiving and Communication System (PACS). Also, the diagnosis information obtaining unit 130 may perform data communication according to a Digital Imaging and Communications in Medicine (DICOM) standard.

The diagnosis information obtaining unit 130 may include one or more elements for enabling communication with an external device. For example, the diagnosis information obtaining unit 130 may include a short-distance wireless communication module, a wired communication module, and a mobile communication module.

The short-distance communication module means a module for short-distance communication within a predetermined distance. Examples of the short-distance communication module may include, but are not limited to, wireless LAN, Wi-Fi, Bluetooth, Zigbee, Wi-Fi Direct (WFD), ultra wideband (UWB), infrared Data Association (IrDA), Bluetooth Low Energy (BLE), and Near Field Communication (NFC).

The wired communication module indicates a module for communication using an electrical signal or an optical signal. Examples of a wired communication technology may include a pair cable, a coaxial cable, an optical fiber cable, an Ethernet cable, or the like.

The wireless communication module exchanges a wireless signal with at least one of a base station, an external terminal, and a server in a mobile communication network. Here, the wireless signal may include various types of data according to a voice call signal, a video call signal, or an exchange of text/multimedia message.

The mode determining unit 140 determines a medical information providing mode of the medical information providing apparatus 100. As described with reference to FIG. 3, the medical information providing mode may be determined according to a type of medical information that is output by the medical information providing apparatus 100 via the display unit 160.

That is, the medical information providing mode may include at least selected from the first mode for providing the shield room inside image to the display unit 160, the second mode for providing the diagnosis information to the display unit 160, and the third mode for providing both the shield room inside image and the diagnosis information to the display unit 160. The display may be transparent in a first mode to present a direct shield room inside view to the user or operator, without using any image capturing units 110. In the third mode, the displayed diagnosis information may then be presented in a transparent fashion to still offer the direct shield room inside view (instead of the shield room inside image obtained with one or more than one image obtaining unit 110), resulting in combined or aggregated third display mode of the first and second display modes together, wherein the first and third display modes are in fact based on a transparency effect of the display units, to allow the operator to see a shield room inside view, optionally through displayed diagnosis information, in stead of actually displayed shield room inside images from any image capturing units. In such embodiments, the observable area is enlarged relative to the area of the window, which remains after arranging the display units on or in the window.

In the present embodiment, the mode determining unit 140 may determine the medical information providing mode of the medical information providing apparatus 100 as a "diagnosis" mode with respect to the target object 10. The "diagnosis" with respect to the target object 10 may include all processes that are performed by the medical diagnosis system 30 with respect to the target object 10, and examples of the processes include a process of obtaining medical information about the target object 10, a process of obtaining medical information (e.g., a process of capturing a medical image), a process of providing medical information and personal target object information, a process of ending the provision of the medical information according to feedback from the operator 5, or the like.

In more detail with reference to an MRI system, the "diagnosis" with respect to the target object 10 may include a process of checking whether any particular factor (e.g., whether a contrast medium is used) exists in capturing an MRI image, in consideration of patient information about the target object 10, a process of guiding the target object 10 to be disposed on a diagnosis table of the MRI system, a process of capturing the MRI image, a process of outputting the captured MRI image, or the like. Here, the aforementioned processes are merely examples of the "diagnosis" performed by the medical diagnosis system 30; thus, one or more embodiments of the present disclosure are not limited thereto.

The mode determining unit 140 in relation to the MRI system may determine the medical information providing mode as each of the processes in the MRI system is performed. That is, whenever the "diagnosis" process of the MRI system proceeds, a type of medical information to be output is changed, and then the mode determining unit 140 may determine the medical information providing mode.

For example, when a diagnosis with respect to a new patient proceeds in the MRI system, and the diagnosis information obtaining unit 130 obtains patient information about the new patient, the mode determining unit 140 may determine the medical information providing mode as the second mode or the third mode, so that the display unit 160 may output the patient information as the diagnosis information on at least one of the display units 160.

Accordingly, when the MRI capturing is ended and the diagnosis information obtaining unit 130 obtains an MRI image, the mode determining unit 140 may determine the medical information providing mode, which was the first mode, to become the second mode or the third mode. At the same time, the mode determining unit 140 may maintain the medical information providing mode of the display unit 160 that operates according to the second mode or the third mode and output the patient information. That is, the mode determining unit 140 may separately determine the medical information providing modes of the display units 160.

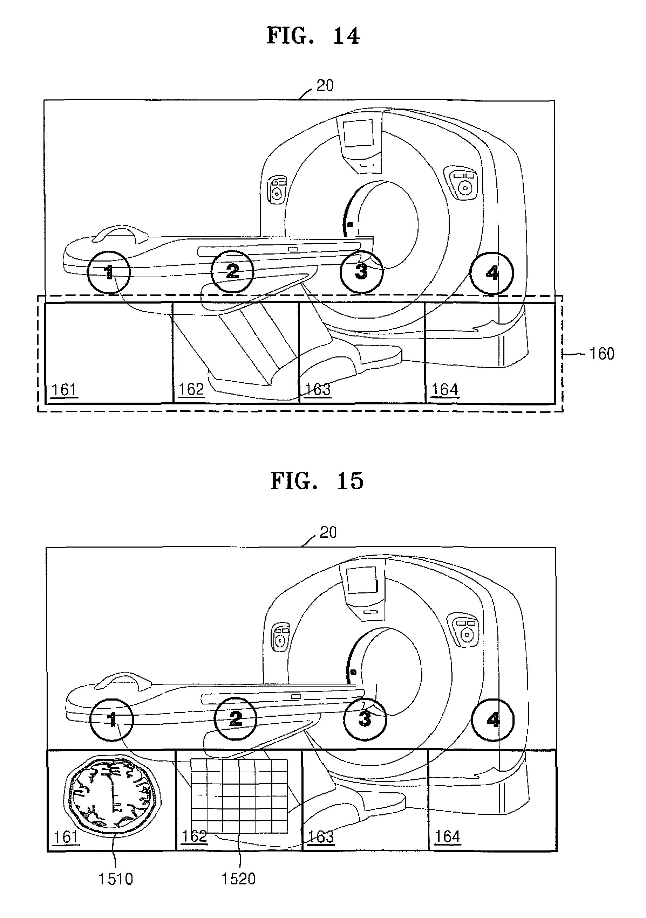

In the present embodiment, the mode determining unit 140 may determine the medical information providing mode according to an increase in a number of pieces of diagnosis information obtained by the diagnosis information obtaining unit 130. In another embodiment, the mode determining unit 140 may determine the medical information providing modes of the display units 160 according to priority orders set in the display units 160. This will be described in detail with reference to FIGS. 15 through 19. The user input unit 150 means a unit by which the operator 5 inputs data so as to control the medical information providing apparatus 100. For example, the user input unit 150 may include, but is not limited to, a key board, a mouse, a dome switch, a touch pad (a touch capacitive type touch pad, a pressure resistive type touch pad, an infrared beam sensing type touch pad, a surface acoustic wave type touch pad, an integral strain gauge type touch pad, a Piezo effect type touch pad, or the like), a jog wheel, a jog switch, or the like. In particular, when the touch pad and a display panel form a mutual layer structure, this structure may be a touch screen. It is evident that for instance in Fig. 2 a single key-board is shown. In the case of an MR / CT console room space utilization can be maximized, relative to the related art in Fig. 1, wherein each monitor or screen is connected to an associated computer or the like, having individual input means, such as keyboards and mouses. According to embodiments of the present disclosure, multiple conventional monitors, screens or display units can be combined and even integrated, to be driven by a single computer or the like, needing only a singular user input unit 150 or only one set thereof (mouse + key-board). Therein, the window and the at least one display unit 160 are driven in combination, i.e. furnished with display data from a single source (the relevant computer or the like

The user input unit 150 may detect not only an actual touch but may also detect a proximate touch. The user input unit 150 may detect a touch input (e.g., a touch & hold input, a tap input, a double-tap input, a flick input, or the like) with respect to the output medical information. Also, the user input unit 150 may detect a drag input from a point in which the touch input is detected. The user input unit 150 may detect multiple touch inputs (e.g., a pinch) with respect to at least two points of the medical information.

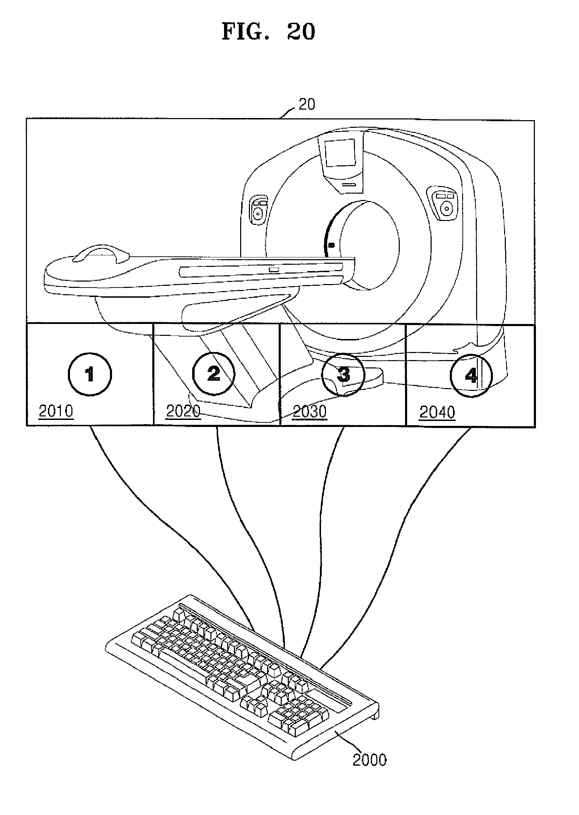

The medical information providing apparatus 100 may include the user input unit 150 that commonly corresponds to the display units 160. The medical information providing apparatus 100 may include a unified user input unit 150 that commonly receives user inputs for controlling the display units 160. This will be described in detail with reference to FIG. 20.

The display unit 160 displays and outputs information that is processed in the medical information providing apparatus 100. The display unit 160 may display the shield room inside image and/or may display the diagnosis information obtained from the medical diagnosis system 30. For example, the display unit 160 may display various information such as the shield room inside image, the medical image, the medical image capturing schedule, or the like. Also, the display unit 160 may overlay the diagnosis information on the shield room inside image and then may display them together.

When the touch pad and the display unit 160 form a layer structure, and thus are formed as a touch screen, the display unit 160 may be used as both an output device and an input device. The display unit 160 may include at least one of a liquid crystal display (LCD), a thin film transistor-liquid crystal display (TFT-LCD), an organic light-emitting display device(OLED), a flexible display, a three-dimensional (3D) display, and an electrophoretic display. According to the way the medical information providing apparatus 100 is embodied, the medical information providing apparatus 100 may include two or more display units 160.

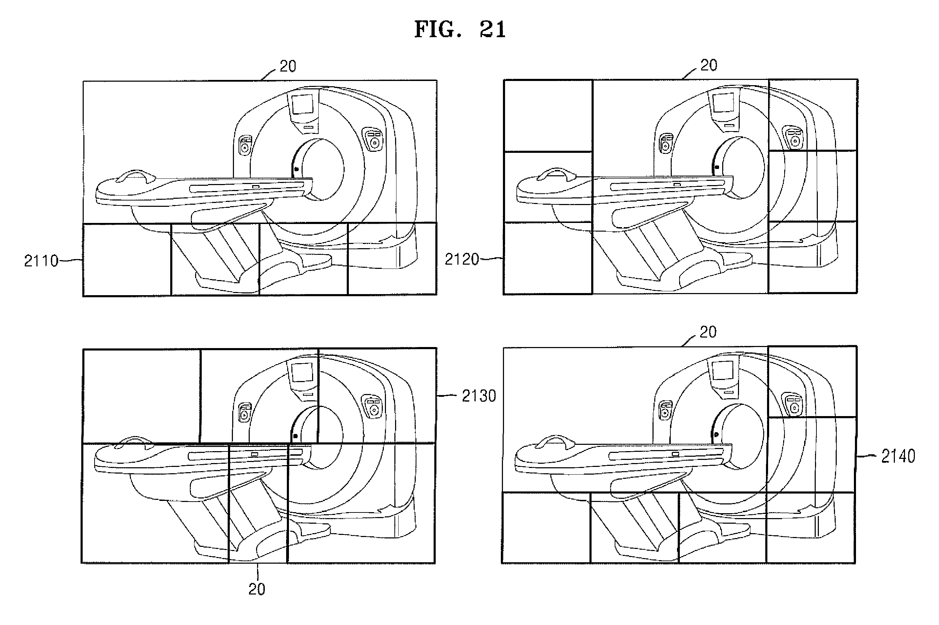

The display unit 160 may be disposed in, on or adjacent to the console room window 20 or a peripheral side thereof. For example, as illustrated in FIG. 2, the display units 160 may be arrayed in one direction adjacent to a lower end of the console room window 20. Alternatively, as illustrated in FIG. 3, the display units 160 may be arrayed in an "L" shape adjacent to right and lower ends of the console room window 20. FIG. 21 shows various arrangements including the aforementioned arrangements.

The display unit 160 may be disposed adjacent to the console room window 20 and may output the shield room inside image. Accordingly, the operator 5 of the medical information providing apparatus 100 may observe an area inside the shield room, wherein the area is bigger and larger than an observation area that the console room window 20 may provide. That is, the medical information providing apparatus 100 may generate an effect by which the console room window 20 is enlarged, and more of the shield room is made visible to the operator on the display units 160 in combination with a remainder of the console room window 20 with the display units on or against this window 20 to allow a direct shield room inside view or display obtained shield room inside images, or with the display units 160 arranged at least partially outside the peripheral circumference of the window 20 to display obtained shield room inside images.

Here, the fact that the display unit 160 is "disposed adjacent to" the console room window 20 may mean that an end or side of the console room window 20 and an end or side of the display unit 160 physically and completely meet each other. However, one or more embodiments of the present disclosure are not limited thereto; thus, the end of the console room window 20 and the end of the display unit 160 may be separated from each other within a predetermined distance by having, but not being limited to, a substance, a structure, or a material disposed there between, wherein the substance, the structure, or the material may shield the console room window 20 from the shield room. The medical information providing apparatus 100 may be embodied so that the predetermined distance may be at a minimum or may additionally be transparent, so as to maximize the effect of enlarging the console room window 20.

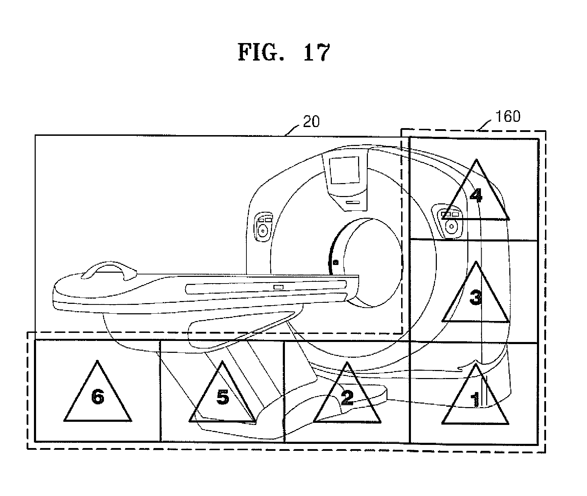

In another embodiment, priority orders may be set in the display units 160. The priority orders may be differently set in the display units 160 or one equal priority order may be set in every two or more display units 160. The display units 160 may be sequentially output diagnosis information according to the set priority orders. This will be described in detail with reference to FIGS. 17 through 19.

The sensor unit 170 in Fig.'s 4 and 9 and 11 senses the operator 5 of the medical information providing apparatus 100. The sensor unit 170 may sense a position of the operator 5, e.g., a distance between the operator 5 and the console room window 20 or between the operator 5 and the display unit 160. In order to sense the operator 5, the sensor unit 170 may include various sensors such as a depth sensor, a distance sensor, a motion recognition sensor, a device recognition sensor, a voice recognition sensor, an ID signal detection sensor, or the like.

The sensor unit 170 may sense not only the operator 5 but also may sense a view or a gesture of the operator 5 by using an iris recognition sensor or a camera. This will be described in detail with reference to FIGS. 9 through 11.

The control unit 180 in Fig. 4 controls all operations of the medical information providing apparatus 100. That is, the control unit 180 may provide, to the display unit 160, the shield room inside image that is captured by the image obtaining unit 110 and/or the diagnosis information that is obtained by the diagnosis information obtaining unit 130. Also, the control unit 180 may provide the medical information to the display unit 160 according to the medical information providing mode. In addition to the aforementioned features, the control unit 180 may control the image processing unit 120 to select a partial region of the shield room inside image based on the sensed user 5, a position thereof in the console room in relation to the shield room, and the like, to present a life like or realistic shield room inside image to the operator 5.

That is, the control unit 180 may generally control operations among the image obtaining unit 110, the image processing unit 120, the diagnosis information obtaining unit 130, the mode determining unit 140, the user input unit 150, the display unit 160, and the sensor unit 170 that are included in the medical information providing apparatus 100.

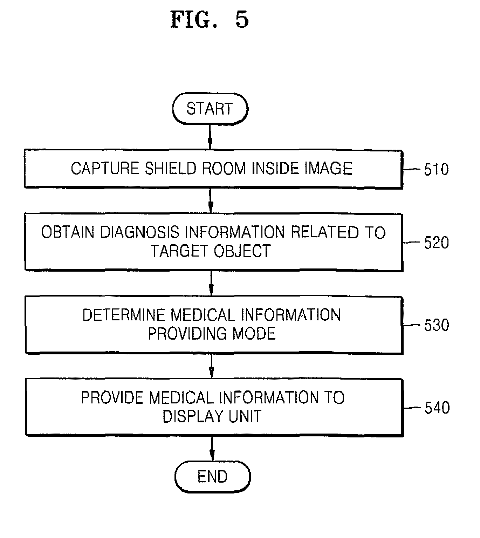

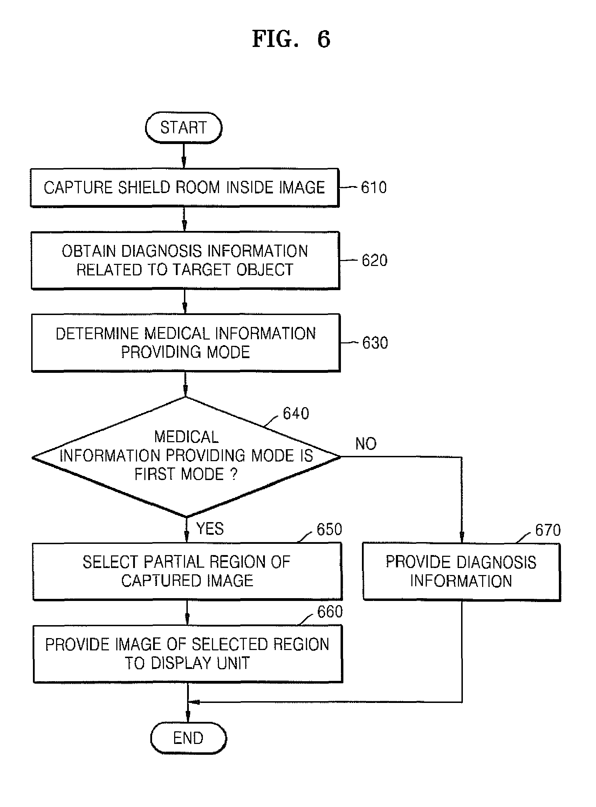

FIG. 5 is a flowchart of a medical information providing method, according to an embodiment of the present disclosure. The flowcharts shown in FIGS. 5 and 6 include operations that are processed in chronological order by the medical information providing apparatus 100, the image obtaining unit 110, the image processing unit 120, the diagnosis information obtaining unit 130, the mode determining unit 140, the user input unit 150, the display unit 160, and the sensor unit 170 that are described with reference to FIG. 4. Thus, hereinafter, although descriptions are omitted, if the descriptions are related to the elements and structures shown in FIG. 4, the descriptions may also be applied to the flowcharts of FIGS. 5 and 6.

In operation 510, the medical information providing apparatus 100 captures a shield room inside image. The medical information providing apparatus 100 may capture the shield room inside image by using at least one image obtaining unit disposed at a back side of a display unit or of a console room window.

In the present embodiment, when a plurality of the display units capture images of the inside of the shield room, the display units could present images of overlapping regions inside the shield room. However, the display units are intended to present images of regions inside the shield room, wherein the regions are separate from each other and preferably augment one another without double displays or omission of parts of regions. This will be described in detail with reference to FIG. 13a and 13b.

In operation 520, the medical information providing apparatus 100 obtains diagnosis information related to a target object. The diagnosis information may be obtained from a medical diagnosis system 30 connected to the medical information providing apparatus 100 via a network. Alternatively, the diagnosis information may be obtained from an external server that is connected to the medical information providing apparatus 100.

The medical information providing apparatus 100 may obtain the diagnosis information that is generated as diagnosis with respect to the target object starts and proceeds. That is, the medical information providing apparatus 100 may obtain new diagnosis information whenever diagnoses with respect to target objects are sequentially performed.

In operation 540, the medical information providing apparatus 100 provides medical information via the display unit 160. In detail, in operation 540, the medical information providing apparatus 100 displays the diagnostic information which is overlaid on the room image according to the third mode, on the display unit 160.

The medical information providing method according to an exemplary embodiment may further include operation 530 after the operation 520. In operation 530, the medical information providing apparatus 100 determines a medical information providing mode. Since the medical information providing apparatus 100 obtains the diagnosis information, the medical information providing apparatus 100 may change a first mode for displaying the shield room inside view through a transparent display unit or the shield room inside image captured by image capturing units 110 and presented o the display units 160 into a second mode or a third mode. That is, after the medical information providing apparatus 100 obtains the diagnosis information, the medical information providing apparatus 100 may change the medical information providing mode that is the first mode, so as to output the diagnosis information to the display unit.

On the contrary, the medical information providing apparatus 100 may change a medical information providing mode of the display unit, which outputs the diagnosis information in the second mode or the third mode, into the first mode. That is, the medical information providing apparatus 100 may change the medical information providing mode so that the display unit may discontinue the output of the diagnosis information and then may output the shield room inside image.

In another embodiment, when the medical information providing apparatus 100 includes a plurality of the display units, the medical information providing apparatus 100 may change medical information providing modes of the display units per display unit, so that the diagnosis information that is displayed on one of the display units may be transferred and displayed on another display unit. That is, the medical information providing apparatus 100 may change the medical information providing mode of each display unit, which displays the diagnosis information, into the first mode, and the medical information providing mode of any other display unit, which is to display the diagnosis information, into the second mode or the third mode.

The second mode and the third mode are distinguished therebetween according to whether or not the display unit displays the shield room inside image along with the diagnosis information. When the medical information providing apparatus 100 displays the diagnosis information on one of the display units, the second mode and the third mode may be determined according to a user input that selects one of the second mode and the third mode. Alternatively, the medical information providing apparatus 100 may select a predetermined mode from among the second and third modes as a default in absence of a user input and then may display the diagnosis information.

In operation 540, the medical information providing apparatus 100 provides medical information via the display unit. As described above, the medical information may include at least one of the shield room inside image and the diagnosis information.

The medical information providing apparatus 100 provides the shield room inside image, the diagnosis information, or both the shield room inside image and the diagnosis information to the display unit, according to the medical information providing mode determined in operation 530. When the medical information providing apparatus 100 includes the display units, the medical information providing apparatus 100 may provide the medical information according to the medical information providing modes that are set in the display units, respectively.

When the medical information providing apparatus 100 obtains new diagnosis information in addition to the medical information displayed on the display unit, the medical information providing apparatus 100 repeatedly performs operations 520 through 540. That is, the medical information providing apparatus 100 may newly determine medical information providing modes of one or more display units according to the new diagnosis information, and may display and output the medical information according to the newly determined medical information providing modes.

FIG. 6 is a flowchart of a medical information providing method, according to another embodiment of the present disclosure. Operations 610 through 630 are similar to operations 510 through 530 that are described with reference to FIG. 5; thus, although descriptions are omitted, if the descriptions are described with reference to FIG. 5, the descriptions may also be applied to operations 610 through 630 of FIG. 6.

In operation 610, the medical information providing apparatus 100 may capture a shield room inside image by using at least one image obtaining unit. In operation 620, the medical information providing apparatus 100 obtains diagnosis information related to a target object. In operation 630, the medical information providing apparatus 100 determines a medical information providing mode of at least one display unit.

In operation 640, the medical information providing apparatus 100 determines whether the medical information providing mode of the display unit is a first mode. When the medical information providing mode of the corresponding display unit is the first mode, the method proceeds to operation 650. When the medical information providing mode of the corresponding display unit is a second mode or a third mode, the method proceeds to operation 670.

In operation 650, the medical information providing apparatus 100 selects a partial region of the shield room inside image that is captured in operation 610. That is, since the medical information providing mode of the display unit to display the medical information is the first mode, the medical information providing apparatus 100 may select the partial region of the shield room inside image. Alternatively, to achieve the first mode, the display unit 160 can be made transparent to present to the operator a direct shield room inside view into the shield room through the transparent display unit in the first mode.

As described with reference to FIG. 4, the medical information providing apparatus 100 may select some of or the entire region of the shield room inside image according to a 1:1 matching relation between the image obtaining unit and the display unit.

In more detail, when the image obtaining unit and the display unit have the 1:1 matching relation, the medical information providing apparatus 100 displays the shield room inside image, which is captured by the image obtaining unit, on the display unit. However, although the image obtaining unit and the display unit have the 1:1 matching relation, the medical information providing apparatus 100 may select a partial region of the shield room inside image by referring to a relation with an operator such as an operator's position, an operator's view, or the like. Also, neighboring display units are to present mutually complementary portions of shield room inside images, preferably without overlap or double representation, for which the selection of partial regions of the shield room inside images obtained by separate image obtaining units associated with distinct display units may also be used. Likewise, appropriate image processing may be applied to present a shield room inside view from an altering perspective of the operator 5, for instance when the operator moves in the console room. The image processing may then be directed at changing the perspective of the shield room inside image, presented on the display unit 160, to correspond with the view that the operator 5 has into the shield room directly through the window 20, which is the shield room inside view.

When the image obtaining unit and the display unit do not have the 1:1 matching relation, the medical information providing apparatus 100 may select a partial region of the shield room inside image and may display an image of the partial region on the display unit. That is, the medical information providing apparatus 100 may select a display-target partial region of the shield room inside image according to an arrangement and position of the display unit. This will be described in detail with reference to FIGS. 12 and 13.

In operation 660, the medical information providing apparatus 100 provides, to the display unit, an image of the region that is selected in operation 650. That is, the medical information providing apparatus 100 may provide the shield room inside image of the region selected in operation 650, by displaying the shield room inside image on the display unit. This step may be omitted in embodiments based on transparent display units 160.

In operation 670, the medical information providing apparatus 100 provides diagnosis information via the display unit. That is, when the medical information providing mode is the second mode or the third mode, the medical information providing apparatus 100 may display and output the diagnosis information that is obtained in operation 620.

In the second mode, the medical information providing apparatus 100 outputs only the diagnosis information to the display unit, whereas the medical information providing apparatus 100 outputs the diagnosis information along with the shield room inside image in the third mode. In the third mode, the medical information providing apparatus 100 may overlap the diagnosis information on the shield room inside image and may display them. In embodiments based on transparent display unit 160, the presentation of the diagnosis information can be made partially transparent, to allow the operator to see directly into the shield room through the display and the diagnosis information displayed thereon.

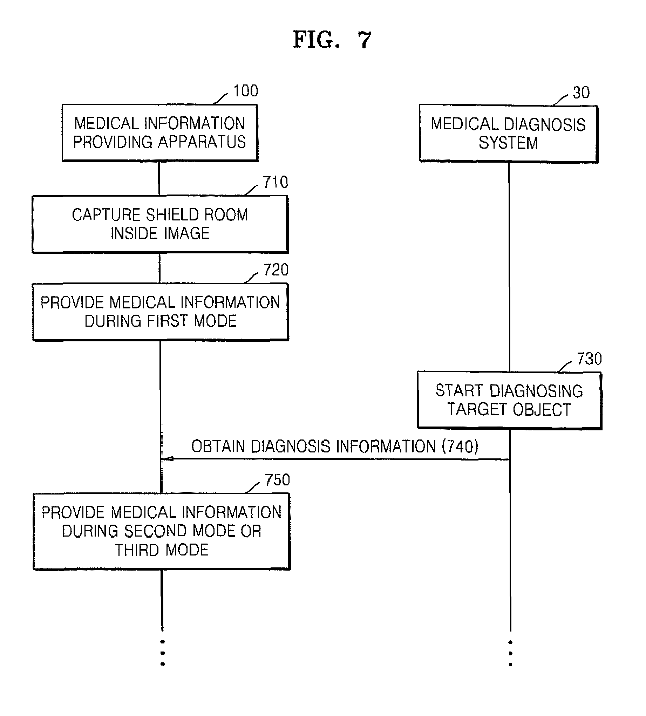

FIG. 7 illustrates operations between the medical information providing apparatus 100 and the medical diagnosis system 30, according to an embodiment of the present disclosure.

In operation 710, the medical information providing apparatus 100 captures a shield room inside image. This operation is the same as that described with reference to FIGS. 5 and 6; thus, detailed descriptions thereof will be omitted here.

In operation 720, the medical information providing apparatus 100 provides medical information during a first mode. That is, the medical information providing apparatus 100 outputs the shield room inside image, which is captured in operation 710, via a display unit.

As described with reference to FIG. 6, the medical information providing apparatus 100 may select a partial region of the shield room inside image and may display an image of the selected partial region on the display unit.

In operation 730, the medical diagnosis system 30 starts diagnosing a target object. That is, the medical diagnosis system 30 performs a diagnosis process with respect to the target object. As described with reference to FIG. 4, the diagnosis process may include all processes that the medical diagnosis system 30 may perform with respect to the target object.

In operation 740, the medical information providing apparatus 100 obtains diagnosis information from the medical diagnosis system 30. The medical information providing apparatus 100 may wiredly or wirelessly receive the diagnosis information from the medical diagnosis system 30 that is connected via a network. In addition to the embodiment of FIG. 7, the medical information providing apparatus 100 may obtain the diagnosis information, such a personal data of the target object, an identification thereof, a treatment plan, a diagnosis plan, and the like, from an external server.

In operation 750, the medical information providing apparatus 100 determines a medical information providing mode as a second mode or a third mode, and then provides the medical information. That is, in order to display the diagnosis information obtained in operation 740, the medical information providing apparatus 100 may change the medical information providing mode from the first mode to the second mode or the third mode.

The medical information providing apparatus 100 may display the diagnosis information on the display unit according to the determined medical information providing mode. As described above, the medical information providing apparatus 100 may display only the diagnosis information in the second mode, and may display both the diagnosis information and the shield room inside image in the third mode, or allow an at least partially transparent presentation of the diagnosis information with a background direct shield room inside view through the display unit.

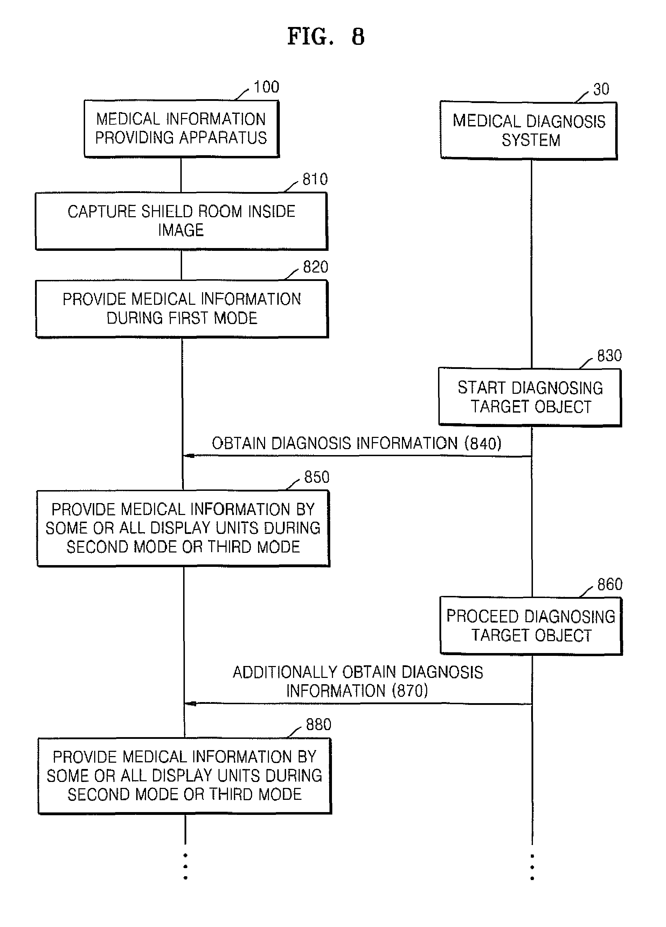

FIG. 8 illustrates operations between the medical information providing apparatus 100 and the medical diagnosis system 30, according to another embodiment of the present disclosure.

In addition to the descriptions with reference to FIG. 7, in the embodiment of FIG. 8, the medical information providing apparatus 100 includes a plurality of display units.

In operation 810, the medical information providing apparatus 100 captures a shield room inside image. In operation 820, the medical information providing apparatus 100 provides the shield room inside image that is captured in operation 810 during a first mode. That is, the medical information providing apparatus 100 of FIG. 8 determines a medical information providing mode of at least one of the display units as the first mode, and displays the shield room inside image captured in operation 810.

In operation 830, the medical diagnosis system 30 starts diagnosing a target object, and in operation 840, the medical information providing apparatus 100 obtains diagnosis information from the medical diagnosis system 30.

In operation 850, the medical information providing apparatus 100 determines the medical information providing mode of at least one of the display units as a second mode or a third mode. Afterward, the medical information providing apparatus 100 may display the diagnosis information on the at least one display unit whose medical information providing mode is determined as the second mode or the third mode.

That is, the medical information providing apparatus 100 may determine medical information providing modes of some or all of the display units as the second mode or the third mode, in consideration of a type of the diagnosis information obtained in operation 840, and a number of diagnosis information to be displayed.

The medical information providing apparatus 100 may determine an order by which the medical information providing modes are changed from the first mode to the second mode or the third mode, based on priority orders that are set in the display units, respectively. That is, the medical information providing apparatus 100 may change the medical information providing modes according to higher priority orders, and may provide the diagnosis information on the display units whose medical information providing modes are changed.

In operation 860, the medical diagnosis system 30 continues the diagnosis with respect to the target object. As a diagnosis process with respect to the target object continues, the medical diagnosis system 30 may additionally generate diagnosis information.

In operation 870, the medical information providing apparatus 100 additionally obtains the diagnosis information that is newly generated. As described above with reference to FIG. 7, the medical information providing apparatus 100 may obtain diagnosis information not only from the medical diagnosis system 30 in operations 840 and 870 but also from an external server that is connected to the medical information providing apparatus 100.

In operation 880, the medical information providing apparatus 100 changes the medical information providing mode of at least one of the display units into the second mode or the third mode, and provides the diagnosis information. That is, in order to display and output the diagnosis information that is newly obtained in operation 870, the medical information providing apparatus 100 may change the medical information providing mode of at least one of the display units.

In operation 880, the medical information providing apparatus 100 may change the medical information providing mode of the at least one display unit, which outputs the diagnosis information during the second mode or the third mode in operation 850, into the first mode. For example, when the medical information providing apparatus 100 discontinues displaying the diagnosis that is obtained in operation 840, and then displays the diagnosis information that is newly obtained in operation 870, the medical information providing apparatus 100 may change the medical information providing mode of the at least one display unit, which outputs the diagnosis information of operation 840, into the first mode, and may change a medical information providing mode of another display unit to output the diagnosis information of operation 870 into the second mode or the third mode.

FIG. 9 illustrates an example in which the medical information providing apparatus 100 senses an operator, according to an embodiment of the present disclosure.

Referring to FIG. 9, a left side and a right side with respect to the console room window 20 at a center indicate a console room and a shield room, respectively. In the embodiment of FIG. 9, the image obtaining unit 110, the display unit 160, and the sensor unit 170 are all disposed adjacent or next to, or in or on the console room window 20. An arrangement of the image obtaining unit 110 and the display unit 160 is described above with reference to FIG. 2.

Referring to FIG. 9, the sensor unit 170 is disposed at a top end of the console room window 20, but one or more embodiments of the present disclosure are not limited thereto. That is, the sensor unit 170 may be disposed at left and right sides of the console room window 20, or may be disposed in the console room, separately from the console room window 20.

As described above with reference to FIG. 4, the sensor unit 170 may include various sensor devices such as the depth sensor, the distance sensor, the motion recognition sensor, the device recognition sensor, the voice recognition sensor, the ID signal detection sensor, the iris recognition sensor, the camera, or the like.

The medical information providing apparatus 100 senses the operator in the shield room. The medical information providing apparatus 100 may sense not only a position and a distance from the display unit by using the sensor device but may also sense the operator by recognizing a specific gesture. Also, the medical information providing apparatus 100 may sense the position and the distance from the display unit by sensing an ID device (e.g., an ID tag or the like) of the operator or by using a signal received from the ID device.

In more detail, the medical information providing apparatus 100 may detect the position of the operator and the distance between the operator and the window 20 or the display unit 160 by using the distance sensor or the depth sensor. Also, the medical information providing apparatus 100 may recognize specific gestures of the operator by using the motion recognition sensor, and may detect a position, in which the specific gesture is detected, as the position of the operator.

Also, the medical information providing apparatus 100 may sense the ID device of the operator. The medical information providing apparatus 100 may detect the ID signal transmitted from the ID device, and may sense the position of the operator from a position of the ID device.

In the present embodiment, when the medical information providing apparatus 100 senses the ID device, the medical information providing apparatus 100 may sense the ID device that is connected to the medical diagnosis system 30. For example, when an operator of the medical information providing apparatus 100 logs into the medical diagnosis system 30 and then diagnoses a target object, an ID device of the operator may include log-in information related to the medical diagnosis system 30. Accordingly, the medical information providing apparatus 100 may sense the operator by detecting the ID device that is logged into the medical diagnosis system 30.

In another embodiment, the medical information providing apparatus 100 may detect a main operator from among a plurality of operators. When the operators are in the console room, the medical information providing apparatus 100 may detect the main operator. For example, when the plurality of operators have ID devices, respectively, the medical information providing apparatus 100 may sense an ID device that is connected to the medical diagnosis system 30 and that is from among the plurality of operators. Accordingly, the medical information providing apparatus 100 may sense the main operator from among the plurality of operators, wherein the main operator logs in the medical diagnosis system 30 and then diagnoses the target object.

In another embodiment, the medical information providing apparatus 100 may sense the main operator by sensing a pre-stored specific gesture. That is, the medical information providing apparatus 100 may recognize the plurality of operators in the console room by using the distance sensor and the depth sensor, and may sense an operator that takes a pre-set gesture and that is from among the plurality of operators, as the main operator.

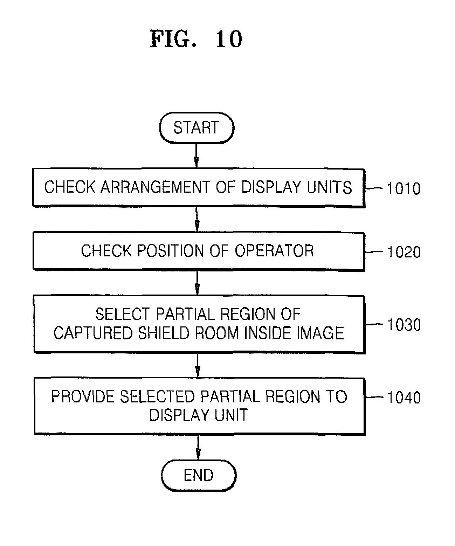

Embodiments with respect to the detection of the operator by the medical information providing apparatus 100 will be described in detail with reference to FIGS. 10 and 11.

FIG. 10 is a flowchart of a method of selecting a partial region of a shield room inside image, according to an embodiment of the present disclosure.

In operation 1010, the medical information providing apparatus 100 checks an arrangement of display units. That is, the medical information providing apparatus 100 may check in which form and positions the display units are disposed adjacent to a console room window 20. The medical information providing apparatus 100 may check the arrangement by receiving pre-stored information about an arrangement of display units 160. Also, the medical information providing apparatus 100 may receive a user input that decides an arrangement and positions of display units, and then may check the arrangement of the display units based on the user input.

In operation 1020, the medical information providing apparatus 100 checks the position of an operator. When the medical information providing apparatus 100 checks the position of the operator, the medical information providing apparatus 100 may sense the operator by employing the various embodiments described above with reference to FIG. 9.

In operation 1030, the medical information providing apparatus 100 selects the partial region of the shield room inside image. This selection may be accompanied by image processing and may be based on a perspective change of the operator looking into the shield room, as indicated above. When the medical information providing apparatus 100 selects the partial region to be displayed on the display unit, the medical information providing apparatus 100 may use a plurality of pieces of information that are checked in operations 1010 and 1020. In more detail, the medical information providing apparatus 100 may check the arrangement and positions of the display units and the position of the operator with respect to the console room window, so that the medical information providing apparatus 100 may effectively generate an effect of enlarging the console room window.

For example, for a case in which the display unit is disposed adjacent to a right side of the console room window, and for a case in which the display unit is disposed adjacent to a left side of the console room window, the medical information providing apparatus 100 has to provide different images to an operator who is positioned at a center in front of the console room window. That is, the medical information providing apparatus 100 may select the partial region of the shield room inside image, in consideration of the arrangement and positions of the display units, and the perspective of the operator looking onto the shield room through the window and at or through the display units 160.

As another example, a view of an operator who is positioned at a left side of the console room window and who watches inside the console room window is different from a view of an operator who is positioned at a right side of the console room window and who watches inside the console room window. Accordingly, the medical information providing apparatus 100 may check an operator's position when the medical information providing apparatus 100 selects the partial region of the shield room inside image which is to be displayed on the display unit.

In operation 1040, the medical information providing apparatus 100 provides an image of the partial region to the display unit. The medical information providing apparatus 100 provides, to the display unit, the image of the partial region that is of the shield room inside image and that is selected in operation 1030. Accordingly, as described with reference to FIG. 2, the medical information providing apparatus 100 may provide the effect of enlarging the console room window with respect to the operator.

FIG. 11 illustrates a method of selecting, by the medical information providing apparatus 100, a partial region of a shield room inside image according to an operator's view, according to an embodiment of the present disclosure.

In the embodiment of FIG. 11, the medical information providing apparatus 100 includes the display units 160 that are horizontally disposed at a lower end of the console room window 20. The medical information providing apparatus 100 may also include the sensor unit 170 that is disposed at a top end of the console room window 20.

First, the medical information providing apparatus 100 captures an image of the inside of a shield room and then obtains a shield room inside image 1120. Afterward, the medical information providing apparatus 100 may sense an operator by using the sensor unit 170. In the present embodiment, the medical information providing apparatus 100 may recognize an operator's eye 1110 by using a view recognition sensor or an iris recognition sensor, and then may sense toward which display unit the operator's eye 1110 is directed.

The medical information providing apparatus 100 may select a partial region 1130 that is of the shield room inside image 1120 and that corresponds to a space toward which the operator's eye 1110 is directed. Then, the medical information providing apparatus 100 provides an image of the partial region 1130 to the display unit 160. Accordingly, the medical information providing apparatus 100 may provide, to the display unit 160, the image of the inside of the shield room which matches the operator's eye 1110.

In the present embodiment, when a plurality of operators are in the console room, the medical information providing apparatus 100 may sense a main operator from among the plurality of operators according to the embodiment of FIG. 9. The medical information providing apparatus 100 may sense a view of the main operator from among the plurality of operators and may output the shield room inside image according to the view of the main operator.

FIG. 12 is a flowchart of a method, performed by the medical information providing apparatus 100, of capturing shield room inside images by using a plurality of image obtaining units, and outputting the shield room inside images to a plurality of display units, according to an embodiment of the present disclosure.

In operation 1210, the medical information providing apparatus 100 captures the shield room inside images by using the plurality of image obtaining units. The plurality of image obtaining units will be described in detail with reference to FIG. 13.

In operation 1220, the medical information providing apparatus 100 matches the plurality of display units with the plurality of image obtaining units. When the medical information providing apparatus 100 includes the same number of the plurality of display units and the plurality of image obtaining units, the plurality of display units and the plurality of image obtaining units may correspond to each other in a 1:1 manner.

On the other hand, the plurality of display units and the plurality of image obtaining units may not match each other in the 1:1 manner. For example, two display units and one image obtaining unit may match each other, or vice versa.

According to the matching relation in operation 1220, the medical information providing apparatus 100 may provide a shield room inside image, which is captured by an image obtaining unit, to a display unit that corresponds to the image obtaining unit. This is described in detail in operations below.

In operation 1230, the medical information providing apparatus 100 checks a position of an operator. Also, in operation 1240, the medical information providing apparatus 100 checks positions of the plurality of display units. Operations 1230 and 1240 may be similar to operations 1010 and 1020 of FIG. 10.

In operation 1250, the medical information providing apparatus 100 determines whether the plurality of display units and the plurality of image obtaining units match each other in the 1:1 manner. When the plurality of display units and the plurality of image obtaining units match each other in the 1:1 manner, the medical information providing apparatus 100 proceeds to operation 1260, and if not, the medical information providing apparatus 100 proceeds to operation 1270.

According to the present embodiment, in operation 1250, the medical information providing apparatus 100 may determine the 1:1 matching relation by using the matching relation of operation 1220.

In operation 1260, the medical information providing apparatus 100 displays the shield room inside image on the matched display unit. That is, the medical information providing apparatus 100 provides the shield room inside images, which are captured by the plurality of image obtaining units in operation 1210, to the plurality of display units that are matched with the plurality of image obtaining units, respectively.

In operation 1270, the medical information providing apparatus 100 selects a partial region of the shield room inside image. That is, when the plurality of display units and the plurality of image obtaining units may not match each other in the 1:1 manner, the medical information providing apparatus 100 selects partial regions of the shield room inside images that are captured by the plurality of image obtaining units, respectively.

That is, the medical information providing apparatus 100 may select the partial regions that are of the shield room inside image captured by the image obtaining units and that are to be provided to the display units that are matched with the image obtaining units, respectively. That is, the medical information providing apparatus 100 may determine images of regions to be displayed on the display units.

The medical information providing apparatus 100 may select the partial regions by using a plurality of pieces of information about the position of the operator and the positions of the display units which are obtained in operations 1230 and 1240. That is, as described above with reference to FIGS. 9 and 10, when the medical information providing apparatus 100 selects the partial regions, the medical information providing apparatus 100 may refer to the arrangement of the display units and a positional relation with the operator.

In operation 1280, the medical information providing apparatus 100 displays the selected images on the matched display units, respectively. The medical information providing apparatus 100 may divide the shield room inside image by referring to a matching relation between the image obtaining units and the display units, and then may display the divided shield room inside images on the display units.

FIGS. 13A and 13B illustrate image obtaining units 112 capturing shield room inside images, according to embodiments of the present disclosure.

In the embodiment of FIG. 13A, the image obtaining units 112 and the display units 160 match each other in a 1:1 manner. In the present embodiment, the medical information providing apparatus 100 includes five image obtaining units 112 and five display units 160. The display units 160 are disposed in one direction at a lower end of the console room window 20, and as described in FIG. 12, the image obtaining units 112 and the display units 160 match each other. Also, in the present embodiment, the image obtaining units 112 may be disposed at back sides of the matched display units 160.

The image obtaining unit 112 captures a shield room inside image of a shield room in which the medical diagnosis system 30 is positioned. That is, the image obtaining units 112 capture shield room inside images, respectively, and the medical information providing apparatus 100 provides the shield room inside images to the matched display units 160. Since the image obtaining units 112 and the display units 160 match each other in the 1:1 manner, the medical information providing apparatus 100 may directly provide the shield room inside images to the display units 160.

The image obtaining unit 112 may capture an image of an overlapping region in the shield room. That is, the image obtaining units 112 may capture overlapping images of the shield room so as to capture an entire region inside the shield room.

Accordingly, the medical information providing apparatus 100 may select partial regions of the shield room inside images that are generated by the image obtaining units 112 and may provide the selected partial regions of the shield room inside images to the display units 160. For example, the medical information providing apparatus 100 may select the partial regions of the shield room inside images, based on a position of a sensed operator, a distance between the operator and the display unit 160, a direction of a view (perspective) of the operator, or the like.

Next, in the embodiment of FIG. 13B, image obtaining units 114 and the display units 160 do not match each other in a 1:1 manner in position or number. In the present embodiment, the medical information providing apparatus 100 includes five display units 160, as in the embodiment of FIG. 13A. On the other hand, the medical information providing apparatus 100 of FIG. 13B includes four image obtaining units 114 that are disposed at four corners of the console room window 20.

Each of the four image obtaining units 114 captures a shield room inside image. Similar to the embodiment of FIG. 13A, the four image obtaining units 114 may capture overlapping images of a shield room so as to capture an entire region inside the shield room.

The medical information providing apparatus 100 may select partial regions of shield room inside images that are captured by the four image obtaining units 114, and then may provide the partial regions to the five display units 160. That is, the medical information providing apparatus 100 may select five partial regions of the shield room inside images captured by the four image obtaining units 114, and may provide images of the five partial regions to the five display units 160, respectively, according to a pre-set matching relation.

FIG. 14 illustrates an example in which the medical information providing apparatus 100 outputs a shield room inside image to a plurality of display units, according to an embodiment of the present disclosure.

In the present embodiment, the medical information providing apparatus 100 includes four display units 160. The four display units 160 are arrayed in one direction at a lower end of the console room window 20. However, a number and arrangement of the display units 160 are not limited to the embodiment of FIG. 14, as is apparent from for instance fig. 13B.

The medical information providing apparatus 100 determines a first mode for medical information providing modes of the four display units 160, and outputs the shield room inside image or drives the display units to be transparent to allow a direct view of the operator into the shield room there through. For convenience of description, the four display units 160 are distinguished from each other by displayed numbers.

The medical information providing apparatus 100 selects four partial regions from the shield room inside image, and provides images of the partial regions to the four display units 160, respectively. The medical information providing apparatus 100 may select the four partial regions so that the four partial regions may be spatially connected to each other, preferably without overlap or missing information there between.

As illustrated in FIG. 14, the partial regions of the shield room inside image which are output via a first display unit 161 and a second display unit 162 are spatially connected to each other. That is, the medical information providing apparatus 100 selects two partial regions that are sequentially connected to each other in the shield room inside image, and outputs images of the sequentially-connected two partial regions to the first and second display units 161 and 162.

Similarly, the partial regions of the shield room inside image which are output to the second display unit 162, a third display unit 163, and a fourth display unit 164 may be sequentially connected to each other. That is, the medical information providing apparatus 100 may provide images of the partial regions selected from the shield room inside image captured with at least one image capturing unit 110 to the display units 160 that are matched with the partial regions, respectively.

As described above, when the medical information providing apparatus 100 outputs the shield room inside image to the display unit 160 that is in the first mode, the medical information providing apparatus 100 may consider a position of an operator, a distance between the operator and the display unit 160, or the like. That is, the medical information providing apparatus 100 may select a partial region to be displayed on the display unit 160, in consideration of a direction of a view of the operator according to the position of the operator while the operator watches the inside of the shield room.

According to the present embodiment, the medical information providing apparatus 100 may allow the operator a view of the inside of the shield room via or through the console room window 20 and additionally the shield room inside image from the image capturing unit(s) and output to the display unit 160, so that the medical information providing apparatus 100 may provide an effect of enlarging the console room window 20 with respect to the operator. In embodiments of transparent displays, the first mode and the third mode may allow the operator to obtain a see through shield room inside view, for which in the first and third modes an at least partially transparent mode of the display unit(s) is set. In the first display mode, in fact no display is achieved, other than to allow the see through effect. Also, the medical information providing apparatus 100 may output images that are connected between the display units 160.

FIG. 15 illustrates an example in which the medical information providing apparatus 100 provides medical information to at least one of the four display units in a second mode or a third mode, according to an embodiment of the present disclosure.

In the embodiment of FIG. 15, the medical information providing apparatus 100 receives diagnosis information from the medical diagnosis system 30. That is, the medical information providing apparatus 100 may capture the shield room inside image according to a medical information providing mode that is a first mode in the embodiment of FIG. 14, and may receive the diagnosis information from the medical diagnosis system 30 or an external server.

In the embodiment of FIG. 15, the medical information providing apparatus 100 obtains a medical image 1510 and a medical image capturing schedule 1520 as the diagnosis information. That is, when a diagnosis with respect to a head of a target object is completed, the medical information providing apparatus 100 may obtain the medical image 1510 of the target object from the medical diagnosis system 30. Similarly, when the diagnosis with respect to the target object is completed, the medical information providing apparatus 100 may obtain the medical image capturing schedule 1520 including information about a next diagnosis target object. Here, the medical image 1510 and the medical image capturing schedule 1520 are examples of the diagnosis information and are for convenience of description; thus, the diagnosis information is not limited thereto.

The medical information providing apparatus 100 may receive the diagnosis information and may determine the medical information providing mode of the display unit 160 as the second mode or the third mode. As described above, the second mode is the medical information providing mode in which the diagnosis information is provided, and the third mode is the medical information providing mode in which the diagnosis information and the shield room inside image are provided together.

In the embodiment of FIG. 15, the medical information providing apparatus 100 provides the medical image 1510 to the first display unit 161, and provides the medical image capturing schedule 1520 to the second display unit 162. An order of providing, by the medical information providing apparatus 100, the diagnosis information to the four display units 160 will be described in detail with reference to FIGS. 17 through 19.

When the medical information providing apparatus 100 determines the medical information providing mode as the second mode or the third mode, the medical information providing apparatus 100 may determine a pre-set medical information providing mode according to a type of the diagnosis information. Alternatively, the medical information providing apparatus 100 receives a user input for selecting a medical information providing mode and may determine the medical information providing mode according to the user input.

For example, when a medical information providing mode with respect to the medical image 1510 is pre-set as the second mode, the medical information providing apparatus 100 may determine a medical information providing mode of a display unit for outputting medical information as the second mode. On the other hand, although a medical information providing mode with respect to the medical image capturing schedule 1520 is pre-set as the second mode, the medical information providing apparatus 100 may receive a user input commanding to overlay the medical image capturing schedule 1520 on the shield room inside image, and thus may output the medical image capturing schedule 1520 in the third mode.

In more detail with respect to the present embodiment, the medical information providing apparatus 100 provides the medical image 1510 to the first display unit 161 in the second mode. The medical information providing apparatus 100 provides the medical image capturing schedule 1520 to the second display unit 162 in the third mode. As illustrated, the second display unit 162 may overlay the medical image capturing schedule 1520 on the shield room inside image so that the second display unit 162 may provide two types of information together.

The medical information providing apparatus 100 may maintain the medical information providing mode that is the first mode for the third display unit 163 and the fourth display unit 164. However, when the medical information providing apparatus 100 obtains new diagnosis information from the medical diagnosis system 30 or receives a user input commanding to transfer the diagnosis information to another display unit 160, the medical information providing apparatus 100 may change the medical information providing mode of the third display unit 163 or the fourth display unit 164 into the second mode or the third mode. That is, the medical information providing apparatus 100 may additionally display the new diagnosis information on the third display unit 163 or the fourth display unit 164.

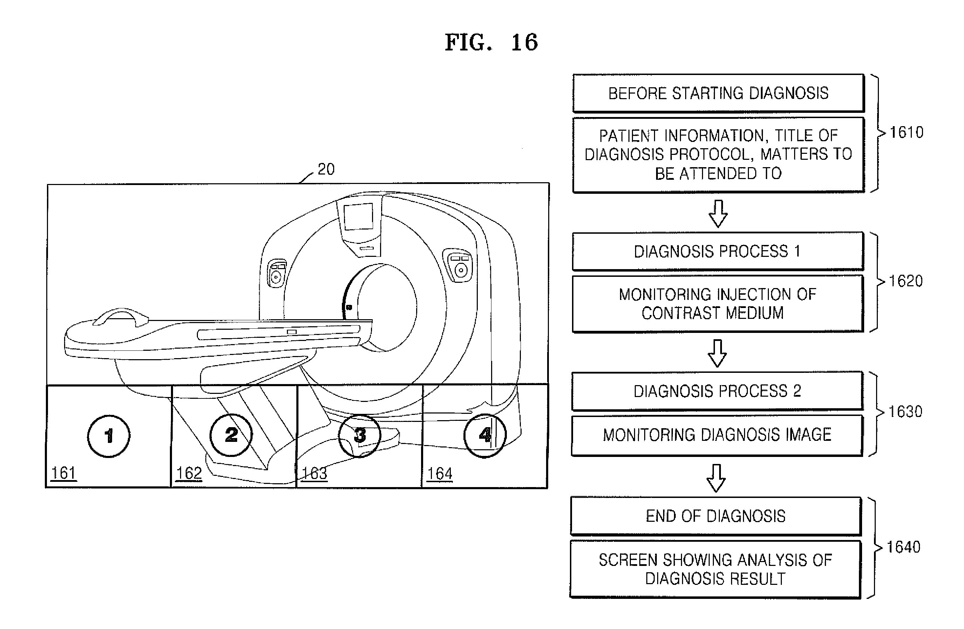

FIG. 16 illustrates a method of changing a medical information providing mode, by the medical information providing apparatus 100, when a diagnosis process with respect to a target object is performed, according to an embodiment of the present disclosure.

As described above with reference to FIGS. 14 and 15, in the present embodiment of FIG. 16, the medical information providing apparatus 100 includes the four display units 160 that are arrayed in one direction at a lower end of the console room window 20. The diagnosis process with respect to a target object, performed by the medical diagnosis system 30, is illustrated at a right side of FIG. 16.

Operation 1610 illustrates a situation before diagnosis starts. Before the diagnosis starts, the medical diagnosis system 30 generates general information such as patient information related to the target object, a medical image capturing schedule, or the like that is used to diagnose the target object.

Accordingly, the medical information providing apparatus 100 may obtain, from for example a hospital server, diagnosis information including the patient information, ID information, information about a diagnosis target part, information about a title of a diagnosis protocol, information about matters to be attended to when diagnosing a patient, or the like.

The medical information providing apparatus 100 may display the diagnosis information, which is obtained in operation 1610, on one or more of the display units 161, 162, 163, and 164. For example, the medical information providing apparatus 100 may display the diagnosis information of operation 1610 on the first display unit 161 in a second or third mode thereof.

Operation 1620 illustrates an ongoing diagnosis process. During the diagnosis process with respect to the target object, the medical diagnosis system 30 may inject a contrast medium into the target object.

In this regard, the medical information providing apparatus 100 may obtain diagnosis information including information about an injection amount of the contrast medium, an injection speed of the contrast medium, a path along which the contrast medium spreads, or the like. Also, the medical information providing apparatus 100 may display the diagnosis information, which is obtained in operation 1620, on one of the display units 161, 162, 163 or 164. For example, the medical information providing apparatus 100 may display the diagnosis information of operation 1620 on the second display unit 162. In another embodiment, the medical information providing apparatus 100 may transfer the diagnosis information of operation 1610 from the first display unit 161 to the second display unit 162 and may display it on the second display unit 162, and may display the diagnosis information of operation 1620 on the first display unit 161.

Operation 1630 illustrates an ongoing diagnosis process after the contrast medium is injected. The medical diagnosis system 30 may generate a medical image obtained by capturing an image of the target object by using the contrast medium.

Accordingly, the medical information providing apparatus 100 may obtain diagnosis information including a plurality of the medical images from the medical diagnosis system 30. That is, the medical information providing apparatus 100 may obtain the medical image that indicates a result of a protocol processed in the medical diagnosis system 30, and may display the medical image on the display unit 160.

In the present embodiment, the medical information providing apparatus 100 may display the medical image of operation 1630 on the third display unit 163 or the fourth display unit 164. On the other hand, the medical information providing apparatus 100 may display the medical image on the first display unit 161 or the second display unit 162, and may transfer the diagnosis information that has been displayed on the first display unit 161 or the second display unit 162 to another display unit and may display the diagnosis information on the other display unit.

Operation 1640 illustrates a situation in which the diagnosis is ended. After the diagnosis with respect to the target object is ended, the medical diagnosis system 30 may output an analysis result about the diagnosis. For example, the medical diagnosis system 30 may calculate a size, a length, and a volume value of the diagnosis target part of the target object, and may image a path of a blood vessel into which the contrast medium is injected. Also, the medical diagnosis system 30 may generate an image showing a position in the diagnosis target part which is estimated to have a tumor.

Accordingly, the medical information providing apparatus 100 may obtain diagnosis information including analysis information with respect to the diagnosis result. Also, the medical information providing apparatus 100 may obtain information about a next target object to be captured, and may also obtain information about a medical image capturing schedule. The medical information providing apparatus 100 may display the obtained information on one of the four display units 161, 162, 163 and/or 164.

In the present embodiment, the medical information providing apparatus 100 may obtain a plurality of pieces of diagnosis information as the diagnosis processes with respect to the target object proceed, and may determine the medical information providing mode of the display unit 160. Also, the medical information providing apparatus 100 may determine the medical information providing mode of the display unit 160 as a number of pieces of the diagnosis information obtained from the medical diagnosis system 30 is increased according to new diagnosis information.

Also, the medical information providing apparatus 100 may determine the medical information providing modes of the display units 160, and may provide a plurality of pieces of diagnosis information, which are different from each other, to the display units 160, respectively.

FIG. 17 illustrates priority orders or sequences, that are set in the display units 160 included in the medical information providing apparatus 100, according to an embodiment of the present disclosure.

As described above with reference to FIGS. 4 and 8, the medical information providing apparatus 100 may set priority orders in the display units 160. The priority orders may mean orders or sequences among the display units 160, and the medical information providing apparatus 100 may provide a plurality of pieces of obtained diagnosis information to the display units 160 according to the priority orders or sequences.

The medical information providing apparatus 100 may determine the priority orders in the display units 160, based on various references. For example, the medical information providing apparatus 100 may determine the priority orders by referring to not only a positional relation between the console room window 20 and the display units 160, such as an arrangement of the display units 160, positions of the display units 160, or the like, but also by referring to specifications such as a size, resolution, or the like of the display units 160.

Here, numbers of the six display units 160 shown in FIG. 17 indicate the priority orders that are set by the medical information providing apparatus 100. That is, the six display units 160 may have the priority orders that correspond to the numbers from 1 to 6 in FIG. 17.

In more detail with respect to the present embodiment, the medical information providing apparatus 100 of FIG. 17 may obtain diagnosis information from the medical diagnosis system 30 and may output the diagnosis information to the display unit 160 (with the number "1") having the highest priority order and positioned at a lower right corner.

Then, the medical information providing apparatus 100 may further obtain two types of diagnosis information and may provide the two types of diagnosis information to the two display units 160 (with the numbers "2" and "3") having the second-highest priority order. That is, the priority orders set in the display units 160 may mean orders by which the medical information providing apparatus 100 displays diagnosis information on the display units 160.

In another embodiment, the medical information providing apparatus 100 may set the same priority order in two or more display units 160. That is, as illustrated, the display units 160 with the numbers "4", "5", and "6" may have the same fourth priority order.

In the present embodiment, when the medical information providing apparatus 100 outputs diagnosis information, the medical information providing apparatus 100 may consider not only the priority orders but may also consider a position of an operator or a direction of view of the operator onto the display units 160. That is, since the three display units 160 "4", "5", and "6" in FIG. 17 have the same priority order, the medical information providing apparatus 100 may determine one of the three display units 160 "4", "5", and "6" to display diagnosis information that is obtained by sensing an operator. For example, when the user is positioned on a left side of the console room window 20, the medical information providing apparatus 100 may display the diagnosis information on the display unit 160 having the number "4" that is from among the three display units 160 with the numbers "4", "5", and "6" and that is adjacent to the operator.

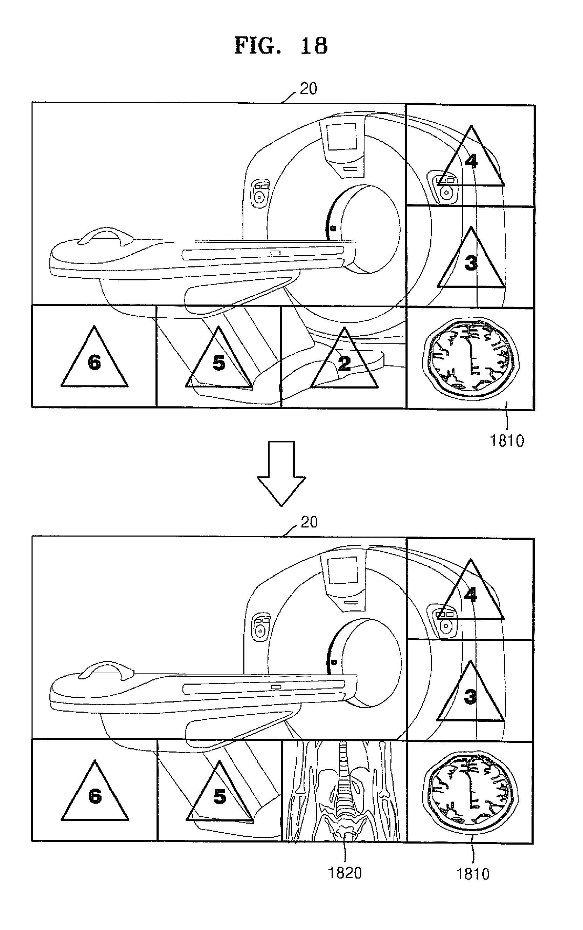

FIG. 18 illustrates an example in which the medical information providing apparatus 100 outputs diagnosis information according to priority orders of display units, according to an embodiment of the present disclosure.

As illustrated in FIG. 17, the medical information providing apparatus 100 may set the priority orders of the six display units. In the embodiment of FIG. 18, different priority orders are set in the six display units, but, as described above, the same priority order may be set in two or more display units.

Referring to an upper diagram of FIG. 18, the medical information providing apparatus 100 obtains, from the medical diagnosis system 30, diagnosis information that is a brain medical image about a brain. The medical information providing apparatus 100 may output the brain medical image, which is the obtained diagnosis information, to a display unit 1810 with a number "1" and having the highest priority order.

Afterward, referring to a lower diagram of FIG. 18, the medical information providing apparatus 100 obtains, from the medical diagnosis system 30, another medical image as diagnosis information that is an abdomen medical image. The medical information providing apparatus 100 may output the abdomen medical image to a display unit 1820 with a number "2" and having the second-highest priority order.

Although not illustrated, when the medical information providing apparatus 100 additionally obtains third diagnosis information from the medical diagnosis system 30, the medical information providing apparatus 100 may display the third diagnosis information on a display unit with a number "3" or any other of the display units 160, depending on the priority order attributed thereto.

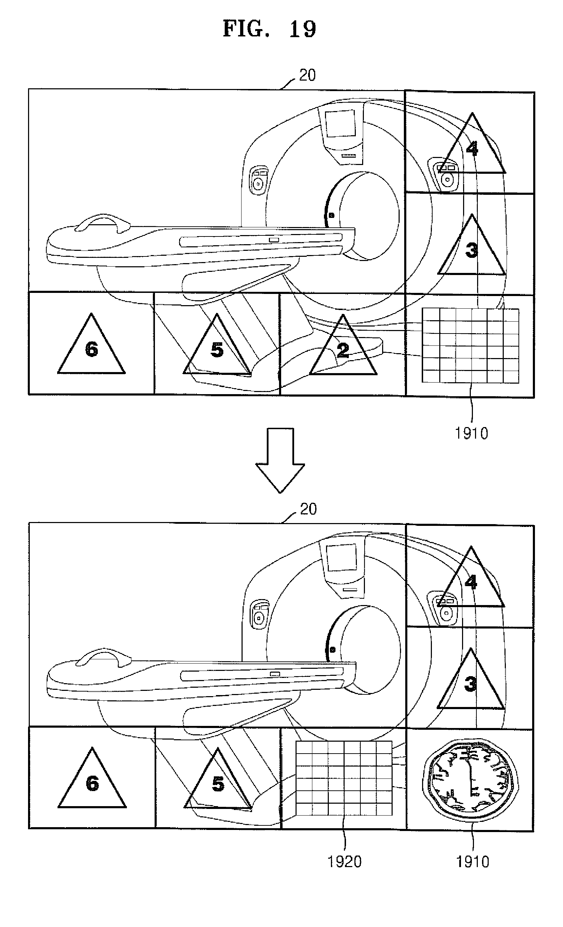

FIG. 19 illustrates an example in which the medical information providing apparatus 100 outputs diagnosis information to display units according to importance of the diagnosis information, according to an embodiment of the present disclosure. In the embodiment of FIG. 19, the medical information providing apparatus 100 may set priority orders in the six display units, wherein the priority orders are the same as the priority orders described with reference to FIG. 17.

Referring to an upper diagram of FIG. 19, the medical information providing apparatus 100 obtains a medical image capturing schedule as diagnosis information from the medical diagnosis system 30. The medical information providing apparatus 100 may change a medical information providing mode of a display unit 1910 having the highest priority order and positioned in a lower right corner from a first mode to a second mode or a third mode. Afterward, the medical information providing apparatus 100 may output the diagnosis information that is the medical image capturing schedule to the display unit 1910 having the changed medical information providing mode.

Referring to a lower diagram of FIG. 19, the medical information providing apparatus 100 obtains a brain medical image as new diagnosis information. As in the embodiment of FIG. 18, although the priority orders are set in the six display units, the medical information providing apparatus 100 may provide information according to importance of the diagnosis information, rather than according to the priority orders of the display units. Alternatively, the priority order may be set depending on the time on which the diagnosis information has become available for display, and the highest priority display unit 1910 is then made available to present the newest diagnosis information.

In more detail, the importance of diagnosis information including a medical image capturing schedule may be lower than the importance of a medical image. That is, the medical information providing apparatus 100 may output diagnosis information to a display unit, in consideration of importance that is pre-set according to a type of the diagnosis information.

In the present embodiment, the medical information providing apparatus 100 may display the medical image, which is diagnosis information having higher importance or is newer or more recently obtained, to the display unit 1910 having the highest priority order and positioned in the lower right corner. In addition, the medical information providing apparatus 100 may transfer and then may display the diagnosis information including the medical image capturing schedule to a display unit 1920 having the second-highest priority order.

That is, when the medical information providing apparatus 100 obtains more pieces of diagnosis information, the medical information providing apparatus 100 may output diagnosis information in consideration of both priority orders of the display units and importance or moment in time of the diagnosis information.

Although not illustrated, when the medical information providing apparatus 100 obtains diagnosis information having importance higher than the medical image, the medical information providing apparatus 100 may transfer and then may output the diagnosis information including the medical image capturing schedule and the medical image to a display unit having a priority order lower than the display unit that has output the diagnosis information including the medical image capturing schedule and the medical image.

FIG. 20 illustrates a user input unit that commonly corresponds to a plurality of display units, according to an embodiment of the present disclosure.

In the embodiment of FIG. 20, the medical information providing apparatus 100 that includes four display units (i.e., the first through fourth display units 2010 through 2040) includes a user input unit 2000 that commonly corresponds to the four display units, but the number of display units is not limited to four. For example, the medical information providing apparatus 100 may include 1, 2, 3, 5, 6, ...10, ...20, etc. display units.

As described above with reference to FIG. 1, a plurality of user input units may be positioned in the console room so as to control the medical diagnosis system 30. When the medical information providing apparatus 100 separately includes a plurality of user input units with respect to a plurality of display units, as in FIG. 1, manipulation convenience may deteriorate and such a configuration is spatially inefficient and economically deficient.

Accordingly, the medical information providing apparatus 100 shown in FIG. 20 may include the user input unit 2000 that corresponds to all of the four display units. The medical information providing apparatus 100 receives a selection input with respect to selecting one of the four display units via the user input unit 2000. Afterward, the medical information providing apparatus 100 may receive a control input with respect to controlling the selected display unit, via the user input unit 2000.

For example, the medical information providing apparatus 100 receives a selection input by which an operator selects the first display unit 2010 from among the four display units. Afterward, the medical information providing apparatus 100 may receive, from the operator, a control input so as to control a shield room inside image or diagnosis information which is output to the first display unit 2010, and may control the first display unit 2010 according to the control input.

The medical information providing apparatus 100 may additionally receive a selection input by which the operator selects the third display unit 2030 from among the four display units. When the medical information providing apparatus 100 receives the selection input and a control input with respect to the third display unit 2030, the medical information providing apparatus 100 may control the third display unit 2030.

The medical information providing apparatus 100 may receive an input as the selection input, wherein the input is generated by pressing a specific key of the user input unit 2000. For example, the medical information providing apparatus 100 may receive user inputs, which are generated by pressing function keys "F1", "F2", "F3", and "F4", as selection inputs that select the first display unit 2010, the second display unit 2020, the third display unit 2030, and the fourth display unit 2040, respectively.

In the present embodiment, after the operator presses the function key "F2", when the operator presses another key of the user input unit 2000, the medical information providing apparatus 100 may detect the second input by the operator as a control input with respect to the second display unit 2020.

FIG. 21 illustrates arrangements of a plurality of display units that are disposed adjacent to the console room window 20, according to embodiments of the present disclosure.

In the embodiment of FIG. 21, the medical information providing apparatus 100 includes the display units that are disposed adjacent to the console room window 20, on a display panel. In detail, the display unit is formed on a display panel such as LDC panel, TFT-LCD panel, OLED panel, etc. In fact the display units are on or against the window 20 at a lower side edge thereof, to allow the camera's on the back of the display units to have a view into the shield room and capture the shield room inside image through the window 20.

In the embodiment at an upper left side of FIG. 21, the medical information providing apparatus 100 may include four display units 2110 that are disposed adjacent to a lower end of the console room window 20. In the embodiment at an upper right side of FIG. 21, the medical information providing apparatus 100 may include six display units 2120 that are disposed adjacent to left and right ends or sides of the console room window 20.

In the embodiment at a lower left side of FIG. 21, the medical information providing apparatus 100 may include five display units 2130 that are disposed adjacent to left, right, and upper ends of the console room window 20. The medical information providing apparatus 100 may include a plurality of display units having different sizes, as in the embodiment at the lower left side of FIG. 21. Larger display units may be attributed a higher priority, as explained above in conjunction with FIG.'s 17 - 19. In the embodiment at a lower right side of FIG. 21, the medical information providing apparatus 100 may include six display units 2140 that form an "L" shape-arrangement and are disposed adjacent to the console room window 20.

In an exemplary embodiment of the medical information providing apparatus 100 may include six display units 2140 that are disposed adjacent to the console room window 20, on the display panel. The medical information providing apparatus 100 may include a plurality of display units having different sizes or equal sizes entirely covering the console room window 20 or leaving a small portion of the console room window 20 uncovered. The entire console window 20 may be covered by a single display unit which may be virtually divided into virtual display units of different size or of equal size, each providing medical or diagnostic information on a separate virtual display unit as described above.

The embodiments shown in FIG. 21 are related to the medical information providing apparatus 100 including the plurality of display units. However, a number of the display units, the arrangements of the display units, and positions of the display units are not limited to the embodiments of FIG. 21.

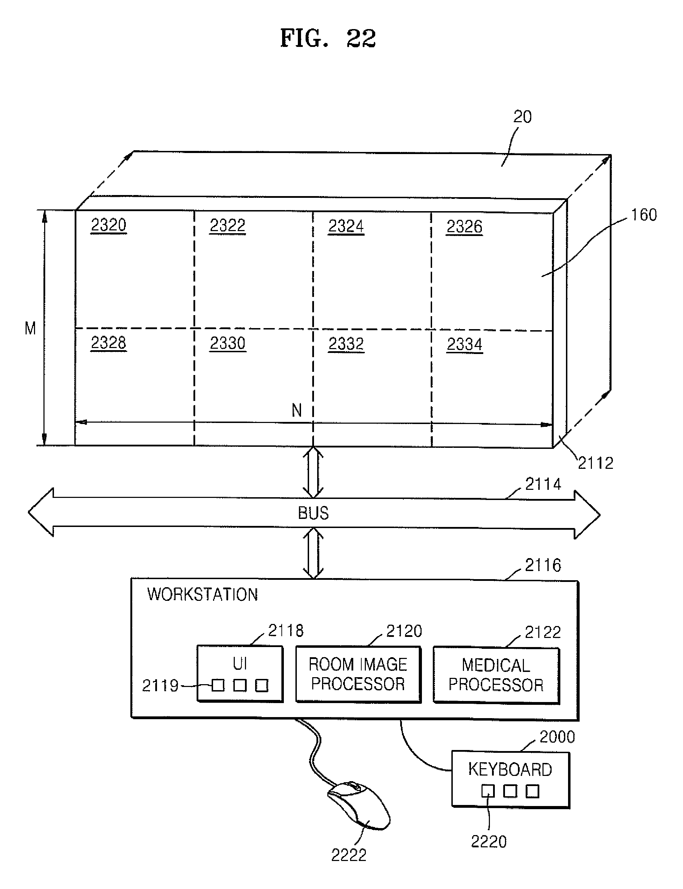

With reference to FIG. 22, the display panel comprising at least one of display may be a separate structure in the console room, may be attached to the console window 20, or may be incorporated in the console window 20. For example, the display units 160 may be formed as an integral structure with the console window 20 or may be fixed to the console window 20 by using adhesives, fixing members such as screws, etc. The structure including the console window 20 and the display units may then be installed between the examination room and the console room. As another example, the display unit may be fixed to the console window 20 which is already installed between the examination room and the console room. The display unit 160 may be arranged in a M x N array and each display or a group of displays may be individually addressed, via a bus 2114, network, or other appropriate wired or wireless connections, by a user operating a workstation 2116. The workstation 2116 may include at least one of a user interface (UI) 2118, a keyboard 2000 including keys 2220, mouse 2222, etc. The UI 2118 may display various graphical objects 2119, menus, screens, etc., so that the user may control the medical examination of the object and the medical equipment disposed in the examination room and also may control the display unit 160.

For example, the user may control the movement of the bed on which the object is disposed, the contrast injection apparatus to inject contrast into the object, a CT scanner and/or MRI system to perform the imaging of the object in the prescribed manner, etc.

For example, the user may control the display unit 160 to define the medical information providing mode, set the priority order, enlarge or reduce the displayed image, move the image from one display to another, etc., by using the UI 2118, the keyboard 2000, the mouse 2222, etc.

For example, the UI 2118 may allow the operator to directly communicate with another professional, i.e., primary care physician, surgeon, etc. For example, when it is noted that an object may need additional diagnostic procedure to be executed, i.e., to image additional target area, to inject a different contrast, to perform a different MR sequence, etc., the operator may directly consult with another medical professional in charge of the object via the UI 2118 and may expeditiously receive the authorization for any further medical procedures for the object.

The workstation 2116 may include a room image processor 2180 configured to obtain the room image from the image obtainers and form a visual representation of the room image and a medical processor 2122 configured to process medical imaging information of the object and form a medical image of a region of interest (ROI) of the object.

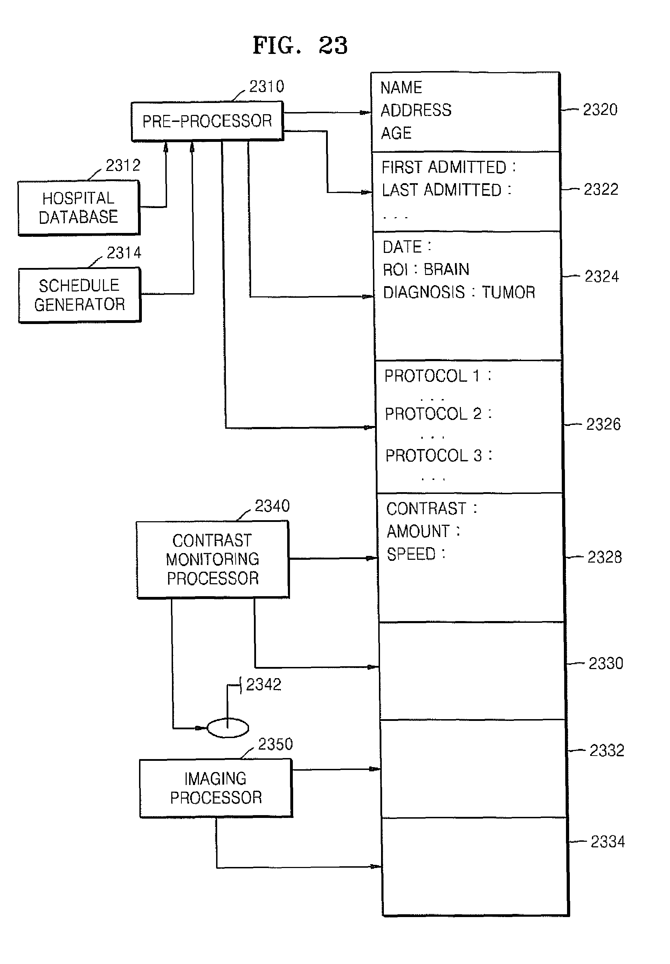

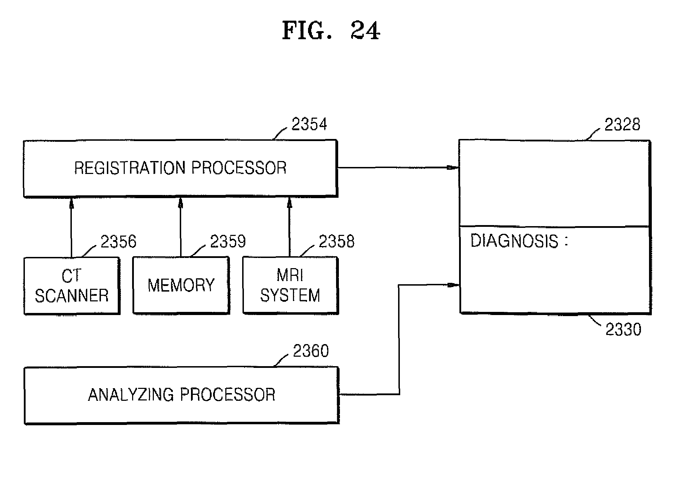

With reference to FIGS. 23 and 24, the medical processor 2122 may include a processor or processors which individually address each of the display unit 160, each of the virtual display unit, or groups of the display unit or virtual display unit.

For example, a pre-processor 2310 may be connected to the hospital database 2312 and may obtain preliminary medical information of the object, as for example, personal records, medical history records, results of the previous imaging procedures, etc., from the hospital database 2312. The pre-processor 2310 may also be connected to a schedule generator 2314 which generates an imaging schedule and/or imaging protocol for the imaging procedure, etc.

The pre-processor 2310 may display each piece of obtained medical information on a separate display unit or on the same display unit. For example, the pre-processor may display the personal records of the object on a first display unit 2320, the medical history records on a second display unit 2322, the results of the previous imaging procedure on a third display unit 2324, and an imaging protocol for the current imaging procedure on a fourth display unit 2326, etc.

The medical information may be displayed in the second medical information providing mode or the third medical information providing mode, while a fifth display unit 2328, a sixth display unit 2330, a seventh display unit 2332, and an eighth display unit 2334 may display the image of the examination room. The order of the display of the medical information may be defined for each display, as described above.

For example, the operation with respect to the pre-processor may correspond to the operation 1610, described above with reference to FIG. 16.

A contrast monitoring processor 2340 may control contrast injection equipment 2342 disposed in the examination room, to inject a contrast medium into the object. For example, the contrast monitoring processor 2340 may obtain and display a contrast name, an injection amount, an injection speed, and a predicted path along which the contrast medium would spread on the fifth display 2328. The contrast monitoring processor 2340 may monitor a path along which the contrast is spreading in the object and display a medical image of the spread of the contrast, on the sixth display unit 2330.

The medical information may be displayed in the second medical information providing mode or the third medical information providing mode, while the seventh display unit 2332 and the eighth display unit 2334 may display the image of the examination room. The order of the display of the medical information may be defined for each display. For example, the sixth display unit 2330, the seventh display unit 2332 and the eighth display unit 2334 may show images of the spread of the contrast at different time points.

For example, the operation with respect to the contrast monitoring processor may correspond to the operation 1620, described above with reference to FIG. 16.

An imaging processor 2350 may obtain and display a medical image of a region of interest (ROI) of the object captured by the imaging apparatus based on the executed imaging protocol, for example, an image of a brain, on the seventh display unit 2332, in the second medical information providing mode or the third medical information providing mode, while the eighth display unit 2334 may display the image of the examination room. The imaging processor 2350 may obtain and display another medical image of another ROI of the object captured by the imaging apparatus based on the executed imaging protocol, for example, an abdomen, on the eighth display unit 2334, in the second medical information providing mode or the third medical information providing mode.

A registration processor 2354 may obtain images of different modalities, for example, from the CT scanner 2356 and the MRI system 2358. Optionally, one of the images may be obtained from a memory 2359. Registration processor 2354 may register the images with one another and display superimposed image on one of the display units in the second medical information providing mode or the third medical information providing mode. For example, some of the previously displayed pieces of medical information, for example, the contrast related information and/or a contrast spreading image may be replaced with the superimposed image of different modalities.

For example, the operations of the imaging processor and the registration processor may correspond to operation 1630 described above with reference to FIG. 16.

An analyzing processor 2360 may analyze the medical information generated during the imaging of the patient and may output a diagnostic result of an analysis in the second medical information providing mode or the third medical information providing mode. For example, the analyzing processor 2360 may generate an image showing a position in the object which is estimated to have a tumor.

For example, the operation described above with respect to the analyzing processor may correspond to the operation 1640, described above with reference to FIG. 16.

As described above, the order of the display of the information may be defined for each display and may be set in the displays or determined by a user. The order of the displaying information described above is illustrative only and is not limiting. The displays of FIGS. 23 and 24 are illustrated as being arranged in a column, for convenience of the description and this is not limiting.

The embodiments of the present disclosure may be written as computer programs and may be implemented in general-use digital computers that execute the programs using a computer-readable recording medium. In addition, a data structure used in the embodiments of the present disclosure may be written in a computer-readable recording medium through various means. Examples of the computer-readable recording medium include magnetic storage media (e.g., ROM, floppy disks, hard disks, etc.), optical recording media (e.g., CD-ROMs, or DVDs), etc.

While this invention has been particularly shown and described with reference to exemplary embodiments thereof, it will be understood by those of ordinary skill in the art that various changes in form and details may be made therein without departing from the scope of the invention as defined by the appended claims.

It is for instance possible that console room window is formed as a display unit, wherein the display unit placed adjacent to this console room window preferably form the enlarged observation area of the inside of the shield room..

The display unit may further be a transparent or translucent display unit arranged be to be at least partially transparent or translucent, for instance in in a display mode wherein no diagnosis information (including scan images and/or patient data and the like) is provided to (a part of) said display unit. Providing the shield room inside view to such a display unit, for instance in the first or third mode, may then include forming the display unit at least partially transparent. The display units are hereto provided on the control room window, i.e. within the boundaries of said control room window, as depicted for example in fig. 21. In this embodiment, an image obtaining unit for capturing a shield room inside image may be omitted.

The exemplary embodiments should be considered in a descriptive sense only and not for purposes of limitation. Therefore, the scope of the invention is defined not by the detailed description of the invention but by the appended claims, and all differences within the scope will be construed as being included in the present disclosure.

## Claims

1. A medical information providing apparatus comprising:
an image obtaining unit for capturing a shield room inside image of the shield room in which the target object is positioned;
a diagnosis information obtaining unit for obtaining diagnosis information related to a target object positioned in a shield room ; and
at least one display unit for displaying the diagnostic information which is overlaid on the room image according to a first mode.

2. The medical information providing apparatus of claim 1,
a mode determining unit for determining a medical information providing mode as at least one of the first mode for providing the diagnostic information on the room image, a second mode for providing a shield room inside view, and a third mode for providing the diagnosis information, and
further comprising a control unit for providing at least one selected from the shield room inside view and the diagnosis information to at least one display unit, according to the medical information providing mode.

3. The medical information providing apparatus of claim 1, wherein the at least one display unit is disposed at a display panel positioned in the console room which is separated from the shield room.

4. The medical information providing apparatus of claim 1,
Wherein the shield room has console room window for observation of the shield room, and
wherein the at least one display unit is disposed adjacent or next to, or in or on the console room window for enlarging an observation area inside the shield room.

5. The medical information providing apparatus of claim 1, further comprising an image processing unit for selecting a partial region of the shield room inside image, and
wherein an image of the partial region is provided to the at least one display unit.

6. The medical information providing apparatus of claim 5, wherein the image obtaining unit selects the partial region based on at least one of a position of the display unit, a position of an operator, a distance between the display unit and the operator and at least one identification (ID) device for identifying the operator, wherein the image obtaining unit preferably selects the partial region based on the at least one ID device of the operator that logs into a medical diagnosis system for diagnosing the target object.

7. The medical information providing apparatus according to any of the claims 2 through 6, wherein the mode determining unit determines the medical information providing mode while a diagnosis process with respect to the target object is being performed, wherein the mode determining unit preferably changes the second mode to the third mode, or the first mode according to claim 2, when the diagnosis process with respect to the target object starts.

8. The medical information providing apparatus according to any of the claims 2 through 7, wherein a plurality of the display units is provided and the mode determining unit determines the medical information providing mode of each of the plurality of the display units.

9. The medical information providing apparatus of claim 8, wherein the mode determining unit determines the medical information providing mode of each of the plurality of the display units as the third mode, or as the first mode according to claim 2, when a number of pieces of obtained diagnosis information are increased, and/or;
changing the medical information providing mode of each of the plurality of the display units, according to priority orders that are pre-set in the plurality of the display units, wherein the priority orders are preferably determined based on at least one of a time of obtaining medical images, a position, a size, and a resolution of each of the plurality of the display units and/or wherein, when a number of pieces of obtained diagnosis information are increased, the control unit provides medical information, which has been provided to a first display unit, to a second display unit that is lower, in terms of the priority orders, than the first display unit.

10. The medical information providing apparatus of any preceding claim, wherein the at least one display unit comprises a plurality of the display units, which are disposed adjacent to each other along at least one top, bottom or side edge of the console room window.

11. The medical information providing apparatus of claim8, 9 or 10, wherein the image processing unit selects a plurality of partial regions from the shield room inside image according to an arrangement of the plurality of the display units, matches the plurality of partial regions with the plurality of the display units, respectively, and provides images of the plurality of partial regions to the plurality of the matched display units.

12. The medical information providing apparatus according to any of the claims 7 - 11, further comprising a user input unit that commonly corresponds to the plurality of the display units, and
wherein the control unit controls the plurality of the display units based on an external input signal that is received via the user input unit.

13. The medical information providing apparatus according to any of the preceding claims, wherein a medical diagnosis system for diagnosing the target object comprises a magnetic resonance imaging (MRI) system or a computer tomography (CT) system.

14. A medical information providing method comprising:
capturing a room image of an inside of an examination room in which an object is positioned for medical examination;
obtaining diagnosis information related to a target object positioned in a shield room; and
displaying the diagnostic information which is overlaid on the room image according to a first mode, on at least one display unit.
determining a medical information providing mode as at least one of a first mode for providing a shield room inside view, a second mode for providing the diagnosis information, and a third mode for providing the shield room inside image and the diagnosis information;
providing at least one selected from the shield room inside view and the diagnosis information to at least one display unit, according to the medical information providing mode,
wherein the at least one display unit is disposed at a display panel positioned in the console room which is separated from the shield room.

15. A non-transitory computer-readable recording medium having recorded thereon a program which, when executed by a computer, performs the method of claim 14.
